# EUROPEAN PATENT APPLICATION

(11) **EP 4 303 882 A1**
(43) Date of publication of application: **10.01.2024**
(21) Application number: 23171218.3
(22) Date of filing: 03.05.2023
(51) Int. Cl.: G16H 40/00, G16H 40/40, G16H 40/67, G16H 10/60

(54) **GLOBAL INDEXING SYSTEM FOR MAINTAINING PATIENT DATA PRIVACY REQUIREMENTS FOR MEDICAL DEVICES AND ASSOCIATED PATIENTS**

(30) Priority: 07.07.2022 US 202263359130 P; 01.05.2023 US 202318310208
(71) Applicant: Pacesetter, Inc., Sylmar, CA 91342 (US)
(72) Inventor: JIN, Maobing, San Jose, CA (US); GAUR, Seema S, Porter Ranch, CA (US)
(74) Representative: Barker Brettell Sweden AB

(57) **Abstract**

A healthcare system (100, 1200) comprises a central service center (102) having processors (1203), memory (1205) storing program instructions and global device index (120) including database records (202a, 202b, 202c) associated with medical devices (1212). Each record (202a, 202b, 202c) includes a corresponding unique device identifier (204), region code (210) associated with a region having corresponding patient data privacy (PDP) requirements and status indicator (212) indicative of whether the corresponding medical device (1212) is active in the corresponding region. The central service center (102) is configured to: i) manage transfer of patient data between regional systems (104a, 104b, 104c) based on the PDP requirements for the corresponding regional systems; ii) manage the status indicators (212), maintained by the first and second regional systems (104a, 104b), associated with a common medical device (1212); iii) return a registration response indicating either: a) device is registered in another regional system (104b, 104c) or b) device is available for registration; or iv) update the status indicator (212) in the global device index (120) for a device in connection with the regional system.

## Description

### TECHNICAL FIELD

Embodiments of the present disclosure generally relate to methods, devices, and systems for tracking and assigning medical devices, and for maintaining privacy and security protections around data exchanges between healthcare regions, thus adhering to patient data privacy requirements.

### BACKGROUND

A digital healthcare solution must store patient data and data associated with a patient's medical device in the patient's own region while meeting the patient data privacy (PDP) requirements of that region. Regions can be geographically and/or politically identified areas, such as the United States, China, and the European Union, each of which can be a different region having different PDP requirements. PDP requirements are defined by regulations that protect a patient's health information (PHI) and personal identification information (PII).

Patients, however, move from region to region, such as by relocating, or may need to receive medical care in a different region they are temporarily visiting. In some cases, standard of care may be greatly diminished if previous and/or current medical information and device information, stored in another region, is not easily and/or quickly accessible. Language differences, time differences, differences between the PDP requirements, and/or the inability to identify and/or contact a specific clinic or hospital that may store the patient's data are all barriers to successfully and securely transferring patient data stored in one region to another. Further, the data must be secured to prevent unauthorized access and/or viewing of PHI and/or PII, and thus cannot simply be accessed by a clinician, printed, copied, mailed, emailed, verbally shared, etc.

Therefore, the need exists for an integrated system to securely identify a medical device without exposing a patient's personal information, and to facilitate the secure transfer of patient data from one region to another.

### SUMMARY

The present invention is defined in the independent claims. Further embodiments of the invention are defined in the dependent claims.

In accordance with embodiments herein, a healthcare system comprises a central service center having memory and one or more processors. The memory is configured to store program instructions and a global device index that includes database records associated with medical devices. Each of the records includes a corresponding unique device identifier (ID), region code and status indicator. The region code is associated with a region having corresponding patient data privacy (PDP) requirements, and the status indicator is indicative of whether the corresponding medical device is active in the corresponding region. The one or more processors, when executing the program instructions, are configured to at least one of: i) manage transfer of patient data, for a first medical device, between first and second regional systems based on the PDP requirements for the corresponding first and second regional systems; ii) when the first and second regional systems maintain corresponding status indicators associated with a common medical device, communicate with the first and second regional systems to manage the status indicators, maintained by the first and second regional systems, associated with the common medical device; iii) responsive to a registration request, for a candidate device, from the first regional system, return a registration response indicating either: a) the candidate device is registered in another regional system or b) the candidate device is available for registration; or iv) responsive to an un-registration request, for a registered device, from the first regional system, update the status indicator in the global device index for the registered device in connection with the first regional system.

Optionally, the unique ID includes at least one of i) a model number, ii) a serial number, iii) a unique key, iv) a second unique key, or v) a birthdate. Optionally, the global device index is further configured to store, associated with the unique IDs, data that does not include patient information identified by the PDP requirements of the first and second regional systems.

Optionally, the one or more processors is configured to receive the registration request, for the candidate device, from the first regional system. The registration request includes one or more unique ID associated with the candidate device. Responsive to the registration request, the one or more processors is further configured to search, based on the one or more unique ID associated with the candidate device, the global device index to determine whether the candidate device is included in any of the regional systems, and in response to determining that the one or more unique ID is included in the second regional system, transmit a communication to the first regional system to report that the candidate device is included in the second regional system.

Optionally, the one or more processors is configured to receive the registration request, for the candidate device, from the first regional system. The registration request includes one or more unique ID associated with the candidate device. Responsive to the registration request, the one or more processors is further configured to search, based on the one or more unique ID associated with the candidate device, the global device index to determine whether the first medical device is included in any of the regional systems, and in response to determining that the one or more unique ID is not included in any of the regional systems, register, in the global device index, the first medical device in the first regional system.

Optionally, the one or more processors is configured to receive a transfer request from the first regional system, to transfer the first medical device from the first regional system to the second regional system. The transfer request includes one or more unique ID associated with the first medical device. Responsive to the transfer request, the one or more processors is further configured to search, based on the one or more unique ID associated with the first medical device, the global device index to determine whether the one or more unique ID is included in any of the regional systems, and in response to determining that the one or more unique ID is included in one of the regional systems other than the first regional system, transmit a communication to the first regional system to report that the first medical device is found in another regional system.

Optionally, the one or more processors is configured to receive a transfer request from the first regional system, to transfer the first medical device from the first regional system to the second regional system. The transfer request includes one or more unique ID associated with the first medical device. Responsive to the transfer request, the one or more processors is further configured to search, based on the one or more unique ID associated with the first medical device, the global device index to determine whether the one or more unique ID is included in any of the regional systems. In response to determining that the one or more unique ID is not included in any of the regional systems other than the first regional system, the one or more processors is configured to update, in the global device index, the first medical device to a pending status, and transmit a request to the second regional system to transfer an assignment of the first medical device to the second regional system.

Optionally, the one or more processors is configured to receive a transfer request from the second regional system, to transfer the first medical device from the first regional system to the second regional system. The transfer request includes one or more unique ID associated with the first medical device. Responsive to the transfer request, the one or more processors is further configured to search, based on the one or more unique ID associated with the first medical device, the global device index to determine whether the one or more unique ID is included in any of the regional systems. In response to determining that the one or more unique ID is included in the first regional system and is not included in any of the other regional systems, the one or more processors is configured to update the status indicator, in the global device index, to indicate an active status of the first medical device in the second regional system, and update the status indicator, in the global device index, to indicate an inactive status of the first medical device in the first regional system.

Optionally, the one or more processors is configured to receive a transfer request from the first regional system, to transfer the patient data associated with the first medical device from the first regional system to the second regional system. The transfer request includes one or more unique ID associated with the first medical device. Responsive to the transfer request, the one or more processors is further configured to, in response to approving the transfer request, transmit a communication to the first regional system to request that the first regional system transmit the patient data to the second region, wherein the patient data includes at least one piece of protected information identified by the corresponding PDP requirements. Optionally, the patient data is encrypted.

Optionally, the one or more processors is configured to receive a request from the first regional system. The request includes an input token value, and the request is one of the registration request, the un-registration request, an update request, or a transfer request. The request includes one or more unique ID associated with one of the medical devices. Responsive to the request, the one or more processors is further configured to decode the request using the input token value, and return, to the first regional system, at least one of a request failed value, a request completed value, a token value associated with a record in the global device index, the input token value, a not found value, a completed value, or a failed value.

In accordance with embodiments herein, a computer implemented method comprises storing, in memory, a global device index that comprises database records associated with medical devices. Each record includes a corresponding unique device identifier (ID), region code and status indicator. The region code is associated with a region having corresponding patient data privacy (PDP) requirements, and the status indicator is indicative of whether the corresponding medical device is active in the corresponding region. The method utilizes one or more processors that, when executing specific program instructions, perform at least one of: i) managing, utilizing one or more processors of a central service center, a transfer of patient data, for a first medical device, between first and second regional systems based on the PDP requirements for the corresponding first and second regional systems; ii) when the first and second regional systems maintain corresponding status indicators associated with a common medical device, communicating, utilizing the one or more processors, with the first and second regional systems to manage the status indicators, maintained by the first and second regional systems, associated with the common medical device; iii) responsive to a registration request, for a candidate device, from the first regional system, returning, utilizing the one or more processors, a registration response indicating either: a) the candidate device is registered in another regional system or b) the candidate device is available for registration; or iv) responsive to an unregistration request, for a registered device, from the first regional system, updating, utilizing the one or more processors, the status indicator in the global device index for the registered device in connection with the first regional system.

Optionally, the unique ID includes at least one of i) a model number, ii) a serial number, iii) a unique key, iv) a second unique key, or v) a birthdate.

Optionally, in response to receiving, utilizing the one or more processors, a transfer request from the first regional system requesting to transfer registration of the first medical device from the first regional system to the second regional system, wherein the transfer request includes one or more unique ID associated with the first medical device, the managing the transfer of patient data further comprises: i) searching, utilizing the one or more processors, based on the one or more unique ID associated with the first medical device, the global device index to determine whether the one or more unique ID is included in any of the regional systems; and ii) in response to determining, utilizing the one or more processors, that the first medical device is not included in any of the regional systems other than the first regional system: a) updating, utilizing the one or more processors, the status indicator of the database record in the global device index associated with the at least one unique ID to a pending status; and b) transmitting, utilizing the one or more processors, a transfer request associated with the first medical device to the second regional system.

Optionally, in response to receiving, utilizing the one or more processors, a transfer request from the first regional system requesting to transfer registration of the first medical device from the second regional system to the first regional system, wherein the transfer request includes one or more unique ID associated with the first medical device, the managing the transfer of patient data further comprises: i) searching, utilizing the one or more processors, based on the one or more unique ID associated with the first medical device, the global device index to determine whether the one or more unique ID is included in any of the regional systems; and ii) in response to determining, utilizing the one or more processors, that the first medical device is included in the second regional system and is not included in any of the other regional systems: a) updating, utilizing the one or more processors, the status indicator of the database record in the global device index associated with the at least one unique ID to register the first medical device in the first regional system; and b) transmitting, utilizing the one or more processors, an un-registration request associated with the first medical device to the second regional system.

In accordance with embodiments herein, a healthcare system comprises a first regional system that comprises a memory and one or more processors. The memory is configured to store program instructions and a regional patient device database including database records associated with medical devices. Each of the records includes a corresponding unique device identifier (ID) and status indicator. The regional patient device database is associated with a region having corresponding patient data privacy (PDP) requirements, and the status indicator is indicative of whether the corresponding medical device is active in the corresponding region. The one or more processors that, when executing the program instructions, is configured to at least one of: i) transmit or receive patient data, for a first medical device, to or from a second regional system associated with a second region, the patient data based on the PDP requirements for at least one of the first and second regional systems; ii) responsive to an update request, for a registered device, update the status indicator for the registered device; iii) responsive to a registration request, for a candidate device, fail the registration of the candidate device if the candidate device is registered in the regional patient device database; or iv) responsive to an un-registration request, for a registered device, un-register the registered device in the regional patient device database.

Optionally, the one or more processors is configured to transmit a request to a central service center, the request being one of the registration request, the un-registration request, the update request, or a transfer request. The request includes one or more unique ID associated with one of the medical devices and an input token value.

Optionally, the one or more processors is configured to: i) determine that the candidate device is not registered in the regional patient device database; ii) transmit the registration request to a central service center, the registration request including one or more unique ID associated with candidate device; and iii) responsive to a communication indicating that the candidate device is not registered in the second regional system, register the candidate device in the regional patient device database.

Optionally, the one or more processors is configured to i) transmit a transfer request to a central service center to request to transfer the first medical device from the first regional system to the second regional system, wherein the transfer request includes one or more unique ID associated with the first medical device; and ii) responsive to a communication indicating that the first medical device is found in the second regional system, fail the transfer request.

Optionally, the one or more processors is configured to: i) transmit a transfer request to a central service center to request to transfer a medical device from the second regional system to the first regional system, wherein the transfer request includes one or more unique ID associated with the medical device; and ii) responsive to a communication indicating that medical device is found in the second regional system, register the medical device in the first regional system.

### BRIEF DESCRIPTION OF THE DRAWINGS

Figure 1 illustrates a healthcare system that is deployed to multiple regions for the sake of patient data privacy requirements and adherence in accordance with embodiments herein.
Figure 2 shows examples of database records in the global device index of the central service center in accordance with embodiments herein.
Figure 3 shows different status transitions of the medical device in the central service center in accordance with embodiments herein.
Figure 4 shows the process flow for registering a new medical device in the healthcare system of Figure 1 in accordance with embodiments herein.
Figure 5 shows the process flow for requesting a patient device transfer wherein one regional system is requesting to push the patient device registration to another regional system in accordance with embodiments herein.
Figure 6 shows the process flow for requesting a patient device transfer wherein one regional system is requesting to pull the patient device registration from another regional system in accordance with embodiments herein.
Figure 7 shows the process flow for un-registering a medical device in accordance with embodiments herein.
Figure 8 shows a table of information used by the healthcare system of Figure 1 for global unique key mapping with encode functions in accordance with embodiments herein.
Figure 9 illustrates a process flow that utilizes one or more API call to lookup a medical device within the healthcare system of Figure 1 without exposing patient PHI or PII in accordance with embodiments herein.
Figure 10 illustrates a process flow that utilizes one or more API call to accomplish cross-region communications for requests that can also include moving patient data to another region without exposing patient PHI or PII in accordance with embodiments herein.
Figure 11 illustrates a process flow that utilizes one or more API call to update the global device index to reflect a desired change within a regional system without exposing patient PHI or PII in accordance with embodiments herein.
Figure 12A illustrates a healthcare system interfacing with a plurality of networked devices formed in accordance with embodiments herein.
Figure 12B illustrates a distributed healthcare system that can be included in one of the regional systems in accordance with embodiments herein.
Figures 13A and 13B show example identifiers associated with protected healthcare information and personal identification information.

### DETAILED DESCRIPTION

It will be readily understood that the components of the embodiments as generally described and illustrated in the Figures herein, may be arranged and designed in a wide variety of different configurations in addition to the described example embodiments. Thus, the following more detailed description of the example embodiments, as represented in the Figures, is not intended to limit the scope of the embodiments, as claimed, but is merely representative of example embodiments.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, etc. In other instances, well-known structures, materials, or operations are not shown or described in detail to avoid obfuscation. The following description is intended only by way of example, and simply illustrates certain example embodiments.

The methods and systems described herein may employ structures or aspects of various embodiments (e.g., systems and/or methods) discussed herein. In various embodiments, certain operations may be omitted or added, certain operations may be combined, certain operations may be performed simultaneously, certain operations may be performed concurrently, certain operations may be split into multiple operations, certain operations may be performed in a different order, or certain operations or series of operations may be re-performed in an iterative fashion. It should be noted that, other methods and systems may be used, in accordance with an embodiment herein. Further, wherein indicated, the methods and systems may be fully or partially implemented by one or more processors of one or more devices or systems. While the operations of some methods and systems may be described as performed by the processor(s) of one device, additionally, some or all of such operations may be performed by the processor(s) of another device described herein.

### TERMS

The term "encoding" shall mean maintaining data usability and can be reversed by employing the same algorithm that encoded the content.

The term "encryption" shall mean maintaining data confidentiality and requires the use of a key or token in order to return to plain text or other original format.

The term "region" shall mean an area, defined by geography, politics, etc., such as a state, a country, an area, and/or a collection of countries. For example, the United States (US), the European Union (EU) (including at least some of the countries within the EU), and China can each be different regions. Optionally, other areas, such as areas outside the EU, may be included in the EU region, for example. Each region can be associated with a different set of patient data privacy requirements. A region does not have to be physically contiguous, and can include one or more areas physically separate from other portions of the region.

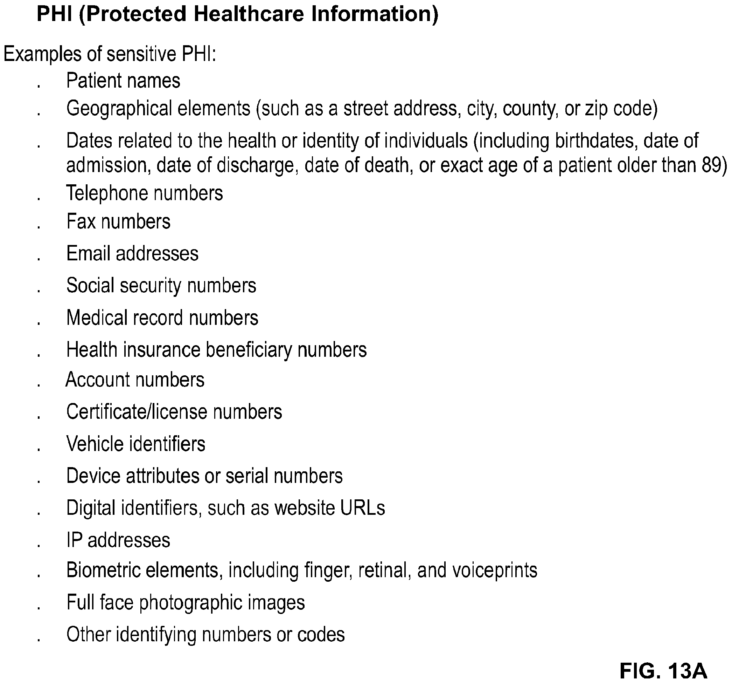

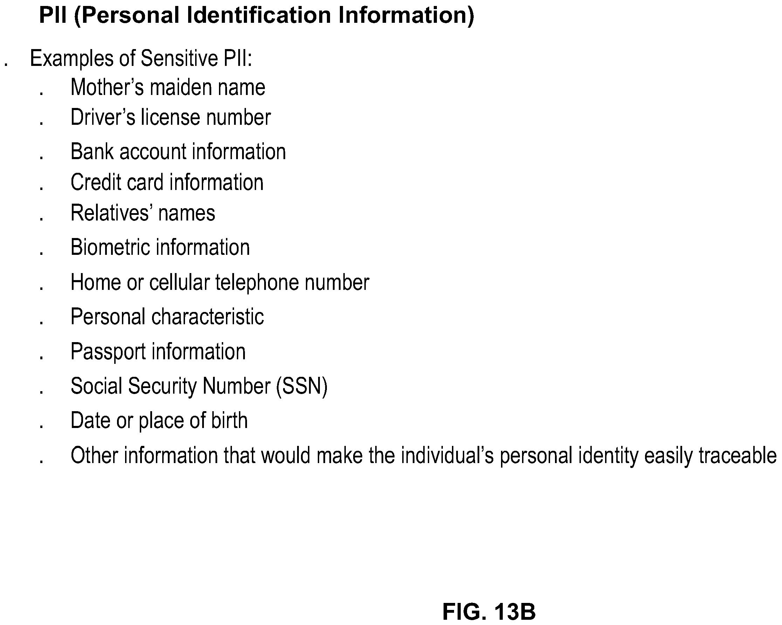

The term "patient data privacy (PDP) requirements" and "PDP" shall mean requirements regarding management and distribution of patient data, where such requirements are defined by a government, state, sovereign entity, agency, administrative body, and the like, The PDP requirements include a plurality of protected healthcare information (PHI) and/or a plurality of personal identification information (PII). The PHI and PII may be different from one region to the next. For example, the US has the Health Insurance Portability and Accountability Act (HIPAA) to protect sensitive patient health information from being disclosed without the patient's consent or knowledge. Within HIPAA, PHI and PII include a number of pieces of private and identifying information that may allow the person/patient to be identified and/or traced. Other countries may have similar privacy laws, such as the General Data Protection Regulation (GDPR) of the EU, the Personal Information Protection and Electronic Documents Act (PIPEDA) of Canada, and the like. The associated patient data cannot be accessed directly by other regions. Although not discussed in detail herein, Figures 13A and 13B include examples of PHI and PII that can be associated with HIPAA. It should be understood that more or fewer identifiers may be included in HIPAA as well as in the PDP requirements of other regions / countries.

The term "patient data" shall mean PHI, PII, as well as data associated with a patient's medical device, such as IMD data and medical device data.

The term "healthcare system" shall mean a system that includes a central service center and two or more regional systems, wherein each of the regional systems is associated with a region. In some cases, a region can have a distributed system such that there are two or more regional systems integrated together but physically separate from each other.

The term "central service center" shall mean a system that includes a patient device index service that includes and accesses a patient device index or database (e.g., centralized index). The database stores information associated with medical devices, such as whether the medical device is registered, and if registered, what region the medical device is registered in, and the status (e.g., active, inactive, pending, open, etc.). The database does not include patient data, although a birth date may be included if used as a key in search terms. The central service center does not belong to any particular region or regional system and has bi-directional communication with the regional systems to process queries, requests, etc., and to synchronize the various databases to keep the medical device information current throughout the healthcare system. Further, the central service center facilitates the exchange of patient data between regions while maintaining the PDP requirements.

The term "regional system" shall mean a patient device service that includes and accesses a patient device database. The system can include a plurality of distributed clinician and/or patient applications for uni- and/or bi-directional communication with the patient device service. The patient device database includes medical device data for the associated region only. The regional system can package, send, and receive patient information associated with one or more medical device to and from the central service center. The regional system can also include and/or interface with one or more data stores that store patient data, IMD data, patient specific device data, medical device data, and the like. Some data stores may be associated with clinicians or groups that can provide data requested by the healthcare system, such as when patient data is to be transferred to another region.

The terms "active", "inactive", "pending", and "open" shall mean the current status of a medical device that is entered into the healthcare system. The term "active" indicates that the medical device is associated with a patient and is in use in an identified region. The term "inactive" indicates that the medical device was active in an identified region but is no longer active, and can also be referred to as a "soft delete". The term "pending" indicates that the status of the medical device is in the process of being changed to another status, and in some cases may be transferred to a different region. The term "open" indicates that the medical device is available (e.g., newly added to the healthcare system, newly manufactured, refurbished, sanitized for the next patient, etc.) to be assigned to a patient within the assigned region, or available to be transferred to another region.

The terms "medical device", "patient device", "candidate device", "medical patient device", "registered device", and/or more generally "device" shall mean any device, implantable (e.g., IMD), semi-implantable, and non-implantable, that acquires and/or transmits medical information/data associated with a patient to a network. A non-implantable device can communicate with one or more implantable device and may also communicate with a network, such as the Merlin@Home^{™} device of Abbott Laboratories (headquartered in the Abbott Park Business Center in Lake Bluff, III.) or another device, such as a Holter monitor. In some cases, a patient can be associated with one or more implantable device, one or more semi-implantable device, and/or one or more non-implantable device.

The terms "unique device identifier" and "unique ID" shall mean one or more unique identifier associated with the medical device. The unique ID can be a device serial number, a device model number, a single code string, a birthdate of a patient, or any other indicator that is associated with the medical device. Each of the medical devices can be associated with one, two or more than two unique IDs.

The term "body generated analyte (BGA) test device" shall mean any and all equipment, devices, disposable products (implantable and/or non-implantable) utilized to collect and analyze a BGA. The BGA test device may implement one or more of the methods, devices and systems as described and referenced to in U.S. Patent Publication Number 2021/0020294 entitled "METHODS, DEVICES AND SYSTEMS FOR HOLISTIC INTEGRATED HEALTHCARE PATIENT MANAGEMENT" published January 21, 2021.

The term "IMD" shall mean an implantable medical device. Embodiments may be implemented in connection with one or more implantable medical devices (IMDs). Non-limiting examples of IMDs include one or more of neurostimulator devices, implantable leadless monitoring and/or therapy devices, and/or alternative implantable medical devices. For example, the IMD may represent a subcutaneous cardioverter defibrillator, cardiac monitoring device, pacemaker, cardioverter, cardiac rhythm management device, defibrillator, cardiac resynchronization device, neurostimulator, leadless monitoring device, leadless pacemaker, left atrial or pulmonary artery pressure sensor, other pressure sensor, blood glucose monitoring device, and the like. The IMD may measure electrical, mechanical, impedance, blood glucose, or pressure information. For example, the IMD may include one or more structural and/or functional aspects of the device(s) described in U.S. Patent Number 9,333,351, entitled "Neurostimulation Method And System To Treat Apnea" issued May 10, 2016 and U.S. Patent Number 9,044,610, entitled "System And Methods For Providing A Distributed Virtual Stimulation Cathode For Use With An Implantable Neurostimulation System" issued June 2, 2015, U.S. Patent Publication Number 2023/0109023, entitled "System and Method for Intra-Body Communication of Sensed Physiologic Data", published April 6, 2023. The IMD may monitor transthoracic impedance, such as implemented by the CorVue algorithm offered by St. Jude Medical. Additionally or alternatively, the IMD may include one or more structural and/or functional aspects of the device(s) described in U.S. Patent Number 9,216,285, entitled "Leadless Implantable Medical Device Having Removable And Fixed Components" issued December 22, 2015 and U.S. Patent Number 8,831,747, entitled "Leadless Neurostimulation Device And Method Including The Same" issued September 9, 2014. Additionally or alternatively, the IMD may include one or more structural and/or functional aspects of the device(s) described in U.S. Patent Number 8,391,980, entitled "Method And System For Identifying A Potential Lead Failure In An Implantable Medical Device" issued March 5, 2013 and U.S. Patent Number 9,232,485, entitled "System And Method For Selectively Communicating With An Implantable Medical Device" issued January 5, 2016. Additionally or alternatively, the IMD may be a subcutaneous IMD that includes one or more structural and/or functional aspects of the device(s) described in U.S. Patent Number 10,765,860, entitled "Subcutaneous Implantation Medical Device With Multiple Parasternal-Anterior Electrodes" issued September 8, 2020; U.S. Patent Number 10,722,704, entitled "Implantable Medical Systems And Methods Including Pulse Generators And Leads" issued July 28, 2020; U.S. Patent Number 11,045,643, entitled "Single Site Implantation Methods For Medical Devices Having Multiple Leads", issued June 29, 2021, U.S. published application US 2021/0330239A1, entitled "Method and system for adaptive-sensing of electrical cardiac signals" filed March 4, 2021. Further, one or more combinations of IMDs may be utilized from the above incorporated patents and applications in accordance with embodiments herein. Embodiments may be implemented in connection with one or more subcutaneous implantable medical devices (S-IMDs). For example, the S-IMD may include one or more structural and/or functional aspects of the device(s) described in U.S. Patent Number 10,722,704, entitled "IMPLANTABLE MEDICAL SYSTEMS AND METHODS INCLUDING PULSE GENERATORS AND LEADS", issued July 28, 2020; U.S. Patent Number 10,765,860, entitled "SUBCUTANEOUS IMPLANTATION MEDICAL DEVICE WITH MULTIPLE PARASTERNAL-ANTERIOR ELECTRODES", issued September 8, 2020. In another example, the IMD may be an ICM that includes one or more structural and/or functional aspects of the device(s) described in U.S. Patent Number 9,949,660, entitled, "Method And System To Discriminate Rhythm Patterns In Cardiac Activity", issued April 24, 2018. The IMD may represent a passive device that utilizes an external power source, and entirely mechanical plan will device, and/or an active device that includes an internal power source. The IMD may deliver some type of therapy/treatment, provide mechanical circulatory support, and/or merely monitor one or more physiologic characteristics of interest (e.g., PAP, CA signals, impedance, heart sounds).

The terms "IMD data" and "medical device data" shall refer to any and all types of information and signals conveyed from an implantable, semi-implantable or external medical device to a local or remote external device. Nonlimiting examples of IMD data include cardiac activity signals (e.g., intracardiac electrogram or IEGM signals), impedance signals (e.g., cardiac, pulmonary or transthoracic impedances), accelerometer signatures (e.g., activity signals, posture/orientation signals, heart sounds), pulmonary arterial pressure signals, MCS rpm levels, MCS flow rates, device alerts, blood glucose level data, hemodynamic stability data, heart rate, blood pressure, pulse oximetry signals, respiratory signals, nerve activity (e.g., as measured within the spinal column or dorsal root), brainwave activity, cholesterol related information, and the like.

The term "MP" shall mean medical personnel, nonlimiting examples of which include doctors, nurses, hospital or clinical staff, pharmacist, physical therapist, and any other person trained or licensed to provide medical assistance to a patient.

The terms "transmit", "transmitting", "obtain" and "obtaining", as used in connection with data, signals, information and the like, shall mean communications between one device and at least one of another device or system, and include at least one of i) accessing memory of an external device or remote server where the data, signals, information, etc., are stored, ii) receiving the data, signals, information, etc., over a wireless communications link between the IMD and/or medical device and a local external device, and/or iii) receiving the data, signals, information, etc., at a remote server over a network connection. The obtaining operation, when from the perspective of an IMD, may include sensing new signals in real time, and/or accessing memory to read stored data, signals, information, etc., from memory within the IMD. The transmitting or obtaining operation, when from the perspective of a local external device, includes receiving the data, signals, information, etc., at a transceiver of the local external device where the data, signals, information, etc., are transmitted from an IMD and/or a remote server. The transmitting or obtaining operation may be from the perspective of a remote server, such as when receiving the data, signals, information, etc., at a network interface from a local external device and/or directly from an IMD. The remote server may also obtain the data, signals, information, etc., from local memory and/or from other memory, such as within a cloud storage environment and/or from the memory of a workstation or clinician external programmer.

The terms "communication", "communications", "query", "application programming interface (API) call", "API call response", and "request" shall be interchangeable and shall mean that uni-directional and/or bi-directional communication is occurring between modules, regional systems, the central service center, medical devices, etc.

The terms "clinician application" and "patient application" shall mean interfaces provided on a plurality of electronic devices that are configured to receive patient data, medical device data, and/or requests that are entered by the patient / clinician. The electronic devices may include, but are not limited to, smart phones, desktop or laptop computers, tablet devices, smart TVs, fixed cameras, smart watch, wearable heart rate monitor, portable or handheld cameras, recording devices, personal digital assistant PDA) devices and the like. The electronic device may include a graphical user interface, through which the patient or another user enters the patient and/or device data. For example, a user may use a keyboard, touch screen, camera, microphone, and/or mouse to enter patient data.

The terms "processor," "a processor", "one or more processors" and "the processor" shall mean one or more processors. The one or more processors may be implemented by one, or by a combination of more than one implantable medical device, a wearable device, a local device, a remote device, a server computing device, a network of server computing devices and the like. The one or more processors may be implemented at a common location or at distributed locations. The one or more processors may implement the various operations described herein in a serial or parallel manner, in a shared-resource configuration and the like.

### System Overview

In accordance with new and unique aspects herein, systems and methods are described that protect confidential patient information while providing the ability to track medical devices globally, update the status of medical devices, add medical devices, and securely transfer patient data from one region to another region. A central service center builds and maintains a global index of all of the medical devices in the healthcare system, but does not retain patient specific information. The central service center can facilitate requests for information, add medical devices, and change the status of medical devices at the global device index as well as all regional systems as needed, thus synchronizing all indexes / databases. The central service center also facilitates the requests for, and transfer of, patient data from one regional system to another regional system without exposing the patient data to any personnel or practitioners.

The systems and methods described herein concern novel uses of, and improvements to, the technology or technical field of the storage and transfer of medical device data and protected patient data related to medical devices while adhering to patient data privacy (PDP) requirements that restrict access to a patient's data. Conventional systems and methods cannot communicate with each other between regions and are not allowed to transfer protected patient data from one region to another. However, patients move, both permanently and temporarily, from a first region to a second region that has different PDP requirements. The patient can require medical care in the second region, and conventional systems provide no solution to transfer the patient's personal protected data from the first to the second region. Therefore, conventional systems require a medical practitioner to directly contact a facility in another region. These conventional methods and systems often experience barriers based on time change, language differences, different PDP requirements, etc., and there is no guarantee that the patient data will be protected as it moves from one region to another.

Systems and methods herein provide the improvements and practical application to integrate regional systems with a central service center that facilitates the secure transfer of data related to medical devices, identifies the region where the medical device is registered, ensures the regional databases and global device index that identify the location and status of medical devices are synchronized with respect to each other, and securely encrypts and transmits patient data between regions. A further improvement is realized as the systems and methods herein identify the location of a medical device and request patient data from and/or push patient data to another region without exposing protected patient data to any personnel. The methods and systems advantageously locate the medical device based on data that is de-identified of patient information and can ensure that the proper paperwork associated with the PDP requirements is acquired from the patient / patient's representative prior to moving the data between regions.

Accordingly, a technical advantage of the systems and methods described herein is the adherence to complicated patient privacy regulations / requirements (e.g., PDP requirements) that are not the same in all regions. A further technical advantage is the ability to securely transfer protected patient information from one region to another to facilitate timely care and accommodate the movement and/or relocation of patients.

### Global Patient Device Indexing Engine

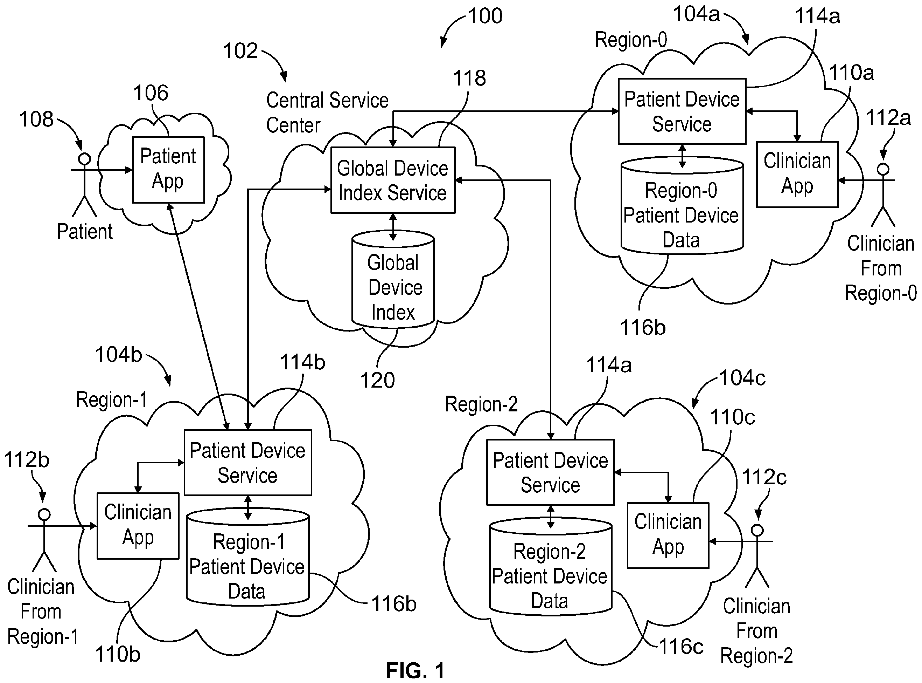

Figure 1 illustrates a healthcare system 100 that is deployed to multiple regions for the sake of patient data privacy (PDP) requirements and adherence in accordance with embodiments herein. The healthcare system 100 provides the ability to query medical device identification data to facilitate several functions without exposing associated protected patient data.

The healthcare system 100 includes the central service center 102 and a plurality of regional systems 104. The central service center 102 includes one or more processors, one or more storage devices (e.g., memory, databases, data lake, etc.), and modem(s)/transmitter(s) to electronically communicate with two or more regional systems 104. Regional system 0 104a may be, for example, located in a first region such as China. Regional system 1 104b can be located in a second region, such as the European Union (EU), while regional system 2 104c can be located in a third region, such as the United States (US). In some embodiments, there can be two regional systems 104, while in other embodiments there can be more than three regional systems 104.

The central service center 102 includes a global device index service 118 (e.g., engine) that can communicate with, and receive and process queries from, the regional systems 104. The global device index service 118 can use the one or more processors to build and access a global device index 120 that provides a centralized index for the healthcare system 100, facilitating the addition of newly entered devices, the modification of existing device records, and the movement of protected patient data and device data from one region to another. The global device index 120 includes at least one of i) one or more unique device identifier (ID) associated with each of the medical devices, ii) the region where the medical device is registered, if applicable, iii) the status of the medical device, iv) when and/or who last updated the medical device record, and v) when and/or who created the medical device record. In some embodiments, the one or more unique ID may be associated with a medical device that includes a plurality of devices, such as an IMD that has two or more separate stimulation and/or monitoring elements. The central service center 102 does not store patient data associated with a medical device (e.g., PHI and/or PII), and can answer queries such as, but not limited to, i) is the medical device data valid, ii) is the medical device registered, iii) is the medical device active, and iv) in which region is the medical device being used. The global device index can be stored as a single database or distributed among different files and/or memory locations while still being integrated together and functioning as a single database and/or index.

The global device index 120 thus indexes the medical devices across all the regional systems 104 without exposing patient privacy. The global device index service 118 manages the global device index 120 to maintain the status of a medical device in each region the device is/was registered in. For example, at any moment in time, only one region should have the medical device in "active" status, and if this is not the case, one or more request associated with the medical device may be rejected.

Each of the regional systems 104 can include one or more processors and modem(s)/transmitter(s) to electronically communicate with a plurality of clinician applications 110 and the central service center 102. The plurality of clinician applications 110 are associated with one regional system 104 and accept input and requests from clinicians 112 located within the same region. For example, the clinician applications 110a accept inputs only from the clinicians 112a located in and/or assigned to the regional system 104a to protect PDP requirements. There can be many clinician applications 110 within the same region, located at doctor offices, hospitals, medical facilities, loaded on a clinician's device (e.g., phone, computer, etc.), and the like. Further, a patient application 106 can be used by a patient 108 to communicate with the regional system 104 that the patient / medical device is assigned to.

In some embodiments, the patient application 106 can transmit medical device data, such as IMD data acquired by their medical device(s), to the clinician application 110, which can forward the data as needed to the patient device service 114 to be transmitted to another region 104.

The regional systems 104 further each include a patient device service 114 (e.g., engine) using the one or more processors to build and/or access a regional patient device database 116 that stores one or more unique ID (e.g., one or more key) that is associated with a particular medical device (e.g., IMD, non-implantable medical device, etc.). The one or more unique ID can be model and serial number, a single code string assigned to a medical device, more than two unique IDs, a model number and a birthdate, and the like. The medical devices can be associated with different venders, and thus the healthcare system is advantageously not limited to medical devices associated with a single vender and is not limited to any particular type of patient care (e.g., cardiac, pulmonary, monitoring elements within blood and/or tissues, etc.). The regional patient device database 116 can also store patient specific information that is protected, such as PHI and PII.

Each of the regional systems 104 includes one or more storage device (e.g., memory, etc.) for storing the regional patient device database 116, which can be stored as a single database or distributed among different files and/or memory locations while still being integrated together and functioning as a single database and/or index. Each of the regional systems 104 has access only to the medical device information assigned to that particular region, and cannot access a different region's patient device database 116 to review or exchange data, and the like.

In some cases, the unique IDs can be uploaded by a clinician to one or more regional system 104 in advance of being assigned to a patient. In other embodiments, the unique IDs can be uploaded by a vender, such as a manufacturing system, singly or in a batch, to the central service center 102 or to a regional system 104 in advance of being assigned to a patient. This has the technical advantage of uploading the known medical device information to the healthcare system 100 while allowing the medical devices to be distributed to any of the regions.

The global device index service 118 provides global application programming interface (API) support for queries from and to the regional systems 104. The one or more unique ID can be entered, for example, by a clinician 112 associated with one of the regional systems 104, and then transmitted in a query to the central service center 102, which stores the unique IDs in the global device index 120. The global device index service 118 can receive and process queries to add a new medical device, register or assign a medical device to a region, inactivate a medical device's association to a region, reassign a medical device to a different region, move patient data associated with a medical device from one region to another, and the like.

The patient device service 114 can also request and receive, such as through the clinician application 110, patient privacy forms if required for data transfer to/from another region. For example, the patient may be required to sign documents associated with PDP requirements associated with the region where the patient data is currently stored and the region where a copy of the patient data is to be transmitted to.

For a data transfer operation, after the central service center 102 has appropriately updated the status of the medical device in each applicable region, the patient device service 114 can package the patient data in a secure encrypted package for transmission to another region. In some cases, the patient device service 114 can request and receive, from the clinician application 110 or other patient data service such as a data repository, hospital data storage system, clinic storage system, etc., medical device data associated with the patient's medical device that is to be transferred to another regional system 104. In some embodiments, a region, such as region 1, can retain patient and device data associated with a patient and/or medical device in an inactive status if the medical device was previously assigned to region 1 and has been transferred to another region and/or deactivated for other reasons (e.g., death, device removal, etc.).

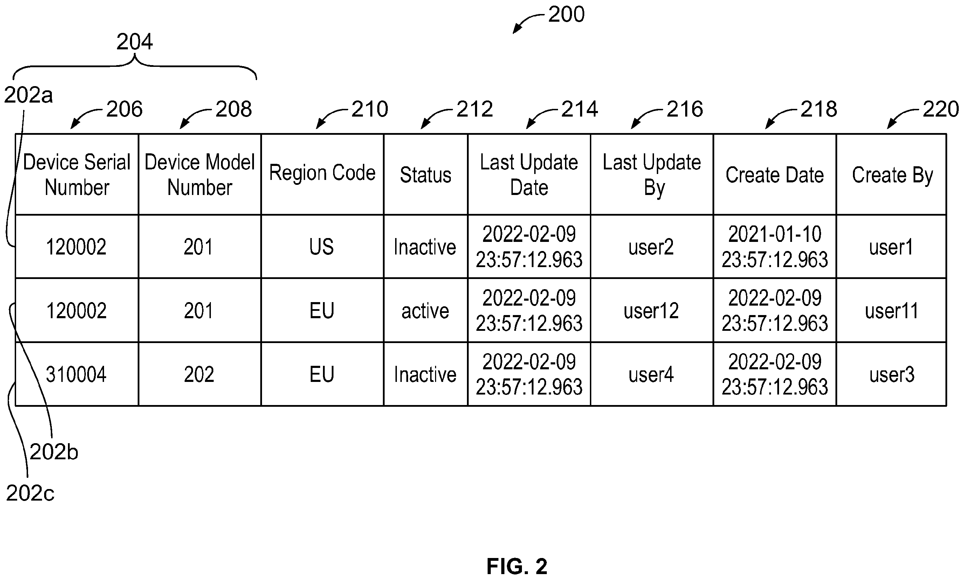

Figure 2 shows examples of database records 200 in the global device index 120 of the central service center 102 in accordance with embodiments herein. It should be understood that more or less fields may be used, depending on what data the global device index service 118 is storing, searching, etc. Historical data can be saved to identify when a medical device is entered into one of the regional systems 104, when the device is updated to change the status, as well as tracking when the update was made and/or by whom.

Three records 202a, 202b, 202c are shown. The columns of the database records 200 as shown include one or more unique ID 204. In Figure 2, the one or more unique ID 204 includes device serial number 206 and device model number 208, although more or less identifiers 204 can be used and are not limited to the types shown. The database records 200 further can include region code 210 (e.g., can be region or any other identifier to be searched), status indicator212 indicating the current status of the medical device, last update date 214 and last update by 216 (e.g., the last clinician 112 to update information associated with the medical device), and create date 218 and create by 220 (e.g., the clinician 112 who originally entered the medical device into the healthcare system 100).

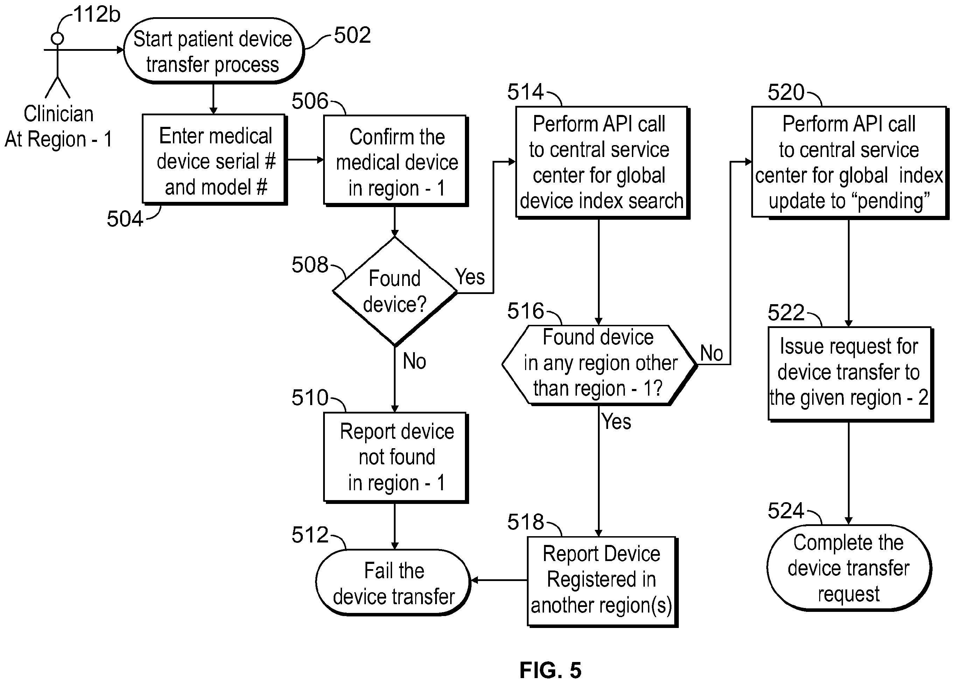

Turning to database records 202a and 202b, these entries are an example of transferring a medical device from a first regional system (e.g., US) to a second regional system (e.g., EU). The process of transferring a medical device from one region to another is further discussed in Figures 5, 6, and 10. The device serial and model numbers 206, 208 (e.g., unique ID(s) 204, key(s), etc.) were originally entered and the record created by user1 on 2021-01-10. Therefore, the device is first registered in the US region. Later, user2 updated the record 202a on 2022-02-09 to change the status indicator 212 of the medical device to inactive in the US (e.g., associated patient may be moving, relocating, needs medical attention, etc., in the EU region), and thus the medical device needs to be reassigned to the EU region. The medical device can only be active in one region at a time. The record 202b indicates that user11 added the medical device in the EU region, and user12 updated the status indicator 212 to "active". In some cases, data associated with the medical device and the patient can be copied, packaged, and securely electronically transferred from the US to the EU via the central service center. The "inactive" status of the record 202a indicates a soft delete, and the data associated with the medical device and the patient can be archived and saved in the regional system 104 for the US region.

The record 202c indicates that user3 added a different medical device in the EU region, and user4 updated the status indicator 212 to "inactive". Although not shown in the database records 200 example of Figure 2, in some cases the medical device can be associated with a single unique ID 204 or key or more than two unique IDs 204. In other cases, the status indicator 212 can be set to "open" if the medical device is available (e.g., newly manufactured, refurbished, sanitized for the next patient, etc.) to be assigned to a patient, or "pending" to indicate that the medical device is in process of a transfer between regions.

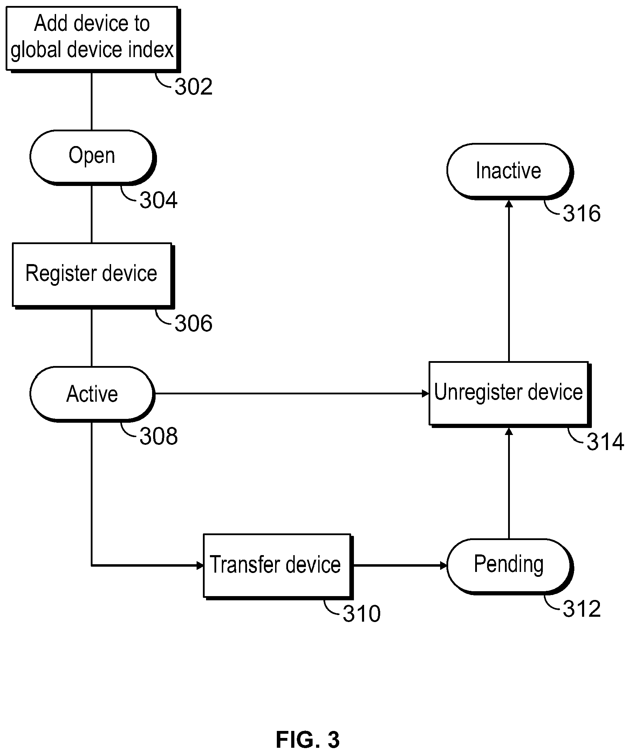

Figure 3 shows different status transitions of the medical device in the central service center 102 in accordance with embodiments herein. It should be understood that one or all of the operations can be accomplished with one or more regional system 104 and/or a combination of the regional(s) 104 and the central service center 102. The operations of Figure 3 may be implemented by hardware, firmware, circuitry and/or one or more processors housed partially and/or entirely within a local external device, remote server or more generally within a healthcare system. For example, the external devices/systems (e.g., local, remote or anywhere within the healthcare system) can include memory and one or more processors.

At 302, one or more processors of the central service center 102 add a medical device to the global device index 120 (e.g., add a database record 202) in response to receiving a communication (e.g., query) including one or more unique ID 204, such as from the clinician application 110a or from a device manufacturer. The communication requests to add a new medical device to the healthcare system 100. The medical device may be a newly manufactured device. For example, a clinician 112a within the regional system 104a (e.g., China) may enter the unique ID(s) 204 for the medical device into the clinician application 110a. The patient device service 114a or other processor(s) transmit the information to the global device index service 118. The one or more processors of the global device index service 118 add the medical device to the global device index 120.

At 304, the one or more processors of the central service center 102 update the status indicator 212 associated with the medical device within the global device index 120 to "open".

At 306, the one or more processors of the central service center 102, in response to receiving the communication from the regional system 104a, register the medical device in the requesting region (e.g., the China region).

At 308, the one or more processors of the central service center 102 change the status indicator 212 from "open" to "active" in the global device index 120. At this point the medical device is associated with a patient in the regional system 104a. However, the global device index 120 includes no personal identifying data associated with the patient.

At 310, the one or more processors of the central service center 102 receive a communication from the regional system 104a or a different regional system 104b, 104c requesting to transfer the medical device to a different regional system 104.

At 312, the one or more processors of the central service center 102 update the status indicator 212 of the medical device to "pending".

At 314, the one or more processors of the central service center 102 receive a communication to un-register the medical device from the currently assigned region. In some embodiments, the medical device may currently be "active", such as at 308, and may be removed from the patient or be deactivated for other reasons. In other embodiments, the patient may have physically relocated to another region, as discussed above with records 202a, 202b of Figure 2.

At 316, the one or more processors of the central service center 102 change the status indicator 212 of the medical device to "inactive" associated with the previously assigned region from either "active" or "pending". If the medical device is being transferred to another region, such as at 310, another record 202 is created for the new region.

### Registering a New Medical Device

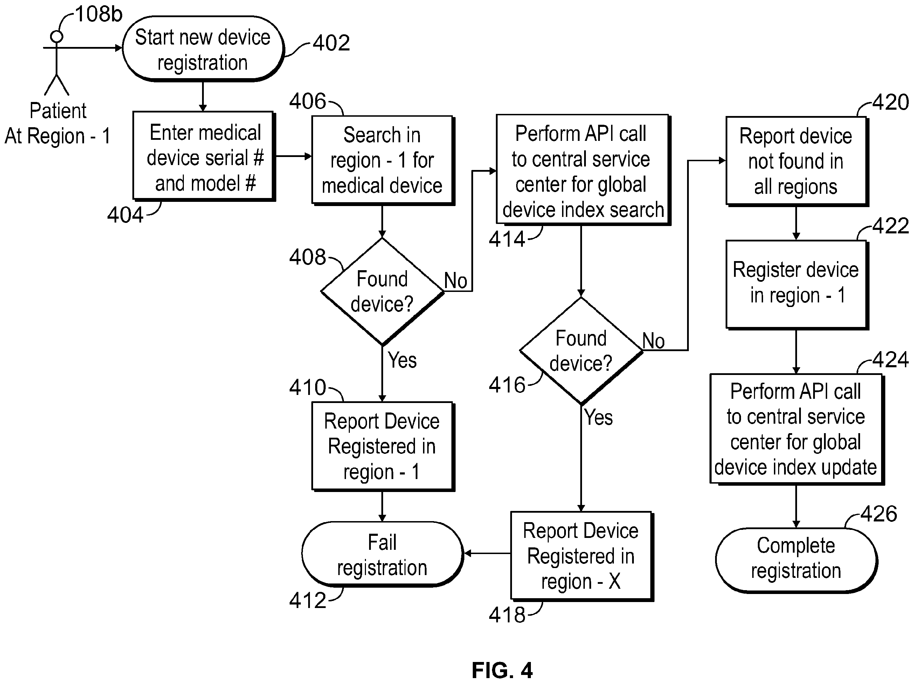

Figure 4 shows the process flow for registering a new medical device in the healthcare system 100 in accordance with embodiments herein. The operations of Figure 4 may be implemented by hardware, firmware, circuitry and/or one or more processors housed partially and/or entirely within a local external device, remote server or more generally within a healthcare system. For example, the external devices/systems (e.g., local, remote or anywhere within the health care system) can include memory and one or more processors.

Figure 4 illustrates a patient 108b within region 1, such as associated with regional system 104b. At 402, one or more processors of the regional system 104b receive a registration request from the clinician application 110b to start a new medical device registration. A new medical device registration is used to register candidate medical devices that have not already been entered into the healthcare system 100.

At 404, the one or more processors of the regional system 104b receive, via the clinician application 110b, the unique ID(s) 204 associated with the medical device. In some embodiments, the device serial number 206 and the device model number 208 are received from the clinician application 110b.

At 406, the one or more processors of the regional system 104b search the patient device database 116b associated with regional system 104b based on the unique ID(s) 204.

At 408, the one or more processors of the regional system 104b determine whether the patient device is entered into the patient device database 116b. If yes, flow passes to 410, where the one or more processors of the regional system 104b report, such as to the clinician application 110b, that the medical device already exists in the patient device database 116b. Accordingly, because the patient device is not a new device, at 412, the one or more processors fail the registration of the medical device. The failure to register can be indicated at the clinician application 110b. This process provides the technical advantage of ensuring that a medical device is not entered more than once into a regional system 104 to prevent any confusion, mis-identification and/or prohibited access to any associated patient data.

Returning to 408, if the medical device is not found in the patient device database 116b, flow passes to 414 and the one or more processors of the regional system 104b perform an API call to query the global device index 120 to determine whether the medical device is or has been registered in any other region.

At 416, if the one or more processors of the central service center 102 determine that the medical device is entered into the global device index 120, flow passes the 418 and one or more processors of the central service center report to the regional system 104b (e.g., transmit a registration response) that the medical device is already registered in a different region. At 412, the one or more processors of the regional system 104b fail the registration of the medical device.

Returning to 416, if the medical device is not found in the global device index 120, flow passes to 420 and the one or more processors of the central service center 102 report to the regional system 104b (e.g., transmit a registration response) that the medical device is not found in any of the regional systems 104 of the healthcare system 100.

At 422, the one or more processors of the regional system 104b register the medical device in the patient device database 116b, indicating the region code 210 to reflect the regional system 104b. In some embodiments, the status indicator 212 of the medical device can be "active" or "open", depending upon whether the device is assigned or not yet assigned, respectively, to a patient.

At 424, the one or more processors of the regional system 104b perform an API call to the central service center 102 to request that a record in the global device index 120 that is associated with the unique ID(s) 204 entered at 404 be created and/or updated to reflect the registration in 422.

At 426, the one or more processors of the central service center 102 complete the registration, such as by updating the global device index 120. In some embodiments, the one or more processors of the central service center 102 can return a message to the clinician application 110b to indicate that the medical device has been updated.

It should be understood that in some embodiments, the new device registration process can be accomplished through the global device index service 118. The patient device service 114 can transfer the request to the central service center 102, and the processor(s) of the global device index service 118 can perform API calls or other communication with one or more region to determine whether the medical device is already registered, and complete the registration if allowed.

### Transfer Medical Device from One Regional System to Another by Pushing

Figure 5 shows the process flow for requesting a patient device transfer wherein one regional system 104 is requesting to push the patient device registration to another regional system 104 in accordance with embodiments herein. The operations of Figure 5 may be implemented by hardware, firmware, circuitry and/or one or more processors housed partially and/or entirely within a local external device, remote server or more generally within a healthcare system. For example, the external devices/systems (e.g., local, remote or anywhere within the health care system) can include memory and one or more processors.

Figure 5 illustrates a clinician 112b within region 1, such as associated with regional system 104b. At 502, one or more processors of the regional system 104b receive a transfer request from the clinician application 110b to start a patient device transfer process for a registered device. For example, a clinician 112b within regional system 104b, such as the EU region, may enter a request into the clinician application 110b to push the medical device data to the regional system 104c, which is associated with the US region.

At 504, the one or more processors of the regional system 104b receive, via the clinician application 110b, the unique ID(s) 204 associated with the medical device. In some embodiments, the device serial number 206 and the device model number 208 are received from the clinician application 110b.

At 506, the one or more processors of the regional system 104b search the patient device database 116b associated with regional system 104b based on the unique ID(s) 204 to confirm that the medical device is a registered device in the regional system 104b.

At 508, the one or more processors of the regional system 104b determine whether the patient device is entered into the patient device database 116b. If no, flow passes to 510, where the one or more processors of the regional system 104b report, such as to the clinician application 110b, that the medical device is not registered in the patient device database 116b. Accordingly, because the patient device is not registered, at 512, the one or more processors of the regional system 104b fail the device transfer of the medical device. The failure to transfer can be indicated at the clinician application 110b.

Returning to 508, if the medical device is found in the patient device database 116b, flow passes to 514 and the one or more processors of the regional system 104b perform an API call to query the global device index 120 to determine whether the medical device is currently "active" in any other region.

At 516, if the one or more processors of the central service center 102 determine that the medical device is "active" in any other region (e.g., a registered device in any other region), flow passes the 518 and the one or more processors of the central service center 102 report, such as to the regional system 104b and the clinician application 11 0b, that the medical device is registered in a different region. In some embodiments, it is prohibited for a medical device to be "active" in more than one region at a time. At 512, the one or more processors of the regional system 104b fail the transfer of the medical device because, in some embodiments, the device transfer request does not conform to the PDP requirements. This provides the technical advantage of preventing personal protected information from being transferred inappropriately.

Returning to 516, if the medical device is only found in the requesting region (e.g., regional system 104b), flow passes to 520 and the one or more processors of the regional system 104b perform an API call to the central service center 102 to request that the status indicator 212 of the record 202 in the global device index 120 associated with the unique ID(s) 204 entered at 504 be updated to "pending". In other embodiments, the status indicator 212 of the record 202 in the patient device database 116b is also updated to "pending".

At 522, the one or more processors of the central service center 102 issue a request, such as to the regional system 104b, to transfer the assignment of the medical device to another region. In this example, the medical device is to be transferred from regional system 104b to regional system 104c.

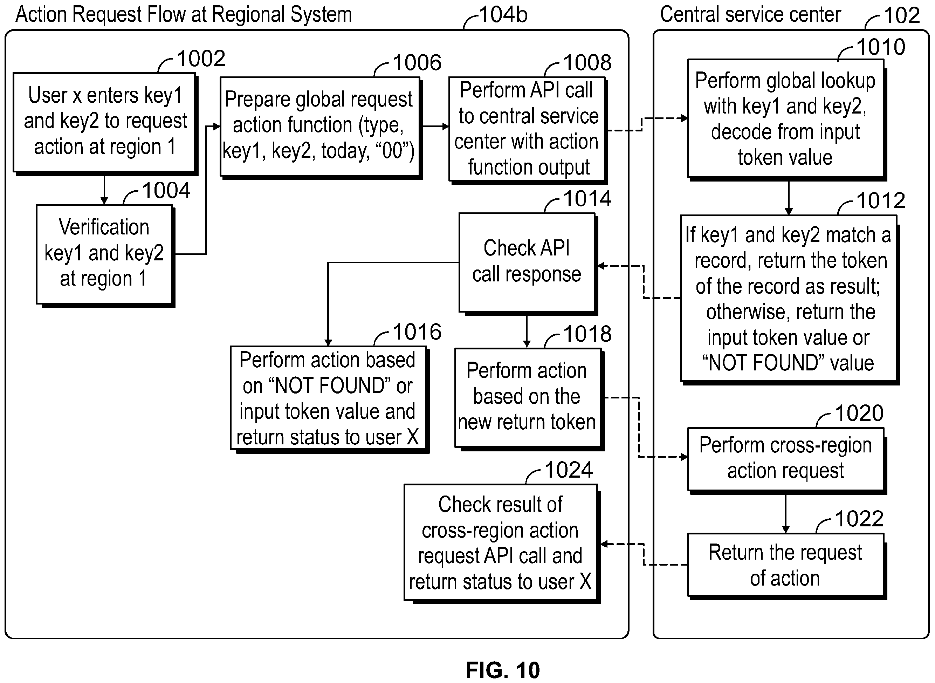

At 524, the one or more processors of the central service center 102 complete the registration, such as by sending a notification of the transfer request to the regional system 104c which may include a request to create a record 202 associated with the medical device in the patient device database 116. Although the transfer request may, in some cases, be generated by the patient device service 114b in the regional system 104b, the transfer request is sent to the regional system 104c via the central service center 102, as direct communication between the different regional systems 104 is not allowed. The transfer of protected information (e.g., PHI, PII, etc.), if needed, between regions is discussed further below in at least Figure 10.

In other embodiments, once the unique ID(s) 204 associated with the medical device are entered at 504, the one or more processors of the central service center 102 can send requests to the appropriate patient device services 114 to determine whether the patient device can be transferred, update the status of the patient device, and complete the device transfer request 524.

### Transfer Medical Device from One Regional System to Another by Pulling

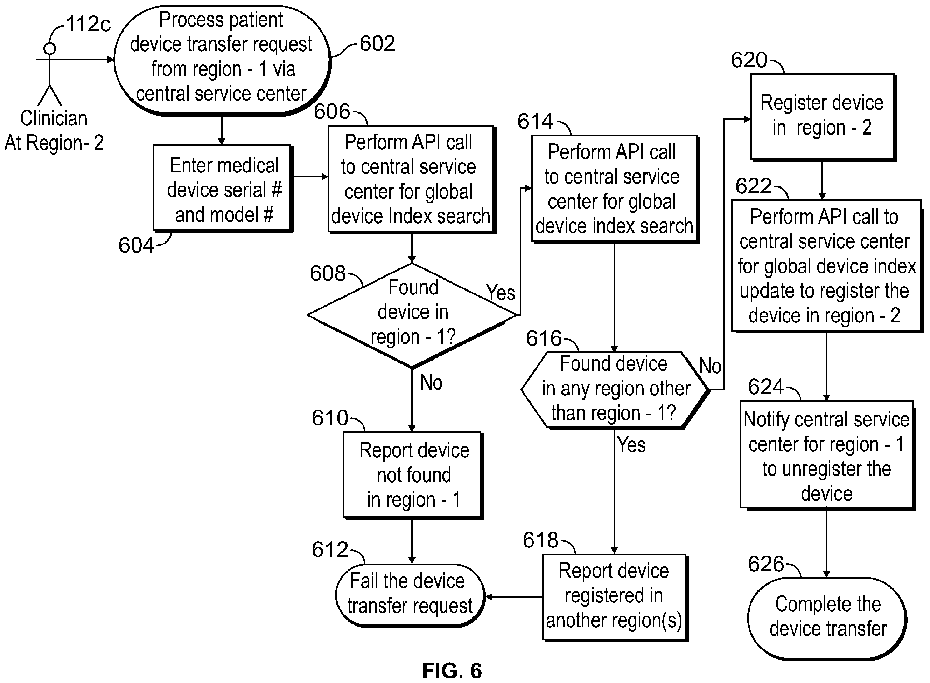

Figure 6 shows the process flow for requesting a patient device transfer wherein one regional system 104 is requesting to pull the patient device registration from another regional system 104 in accordance with embodiments herein. The operations of Figure 6 may be implemented by hardware, firmware, circuitry and/or one or more processors housed partially and/or entirely within a local external device, remote server or more generally within a healthcare system. For example, the external devices/systems (e.g., local, remote or anywhere within the health care system) can include memory and one or more processors.

Figure 6 illustrates a clinician 112c within region 2, such as regional system 104c. At 602, one or more processors of the regional system 104c receive a transfer request from the central service center 102 to process a patient device transfer request from another region. For example, a clinician 112c within regional system 104c, such as the US region, may be responding to a request to pull the medical device registration from the regional system 104b, which is associated with the EU region.

At 604, the one or more processors of the regional system 104c receive, via the clinician application 110c, the unique ID(s) 204 associated with the medical device, such as the device serial number 206 and the device model number 208. In some cases, the unique ID(s) 204 may be provided by the transfer request.

At 606, the one or more processors of the regional system 104c perform an API call to query the global device index 120 to determine whether the medical device is currently found in regional system 104b.

At 608, the one or more processors of the central service center 102 determine whether the medical device is found (e.g., registered) to the regional system 104b in the global device index 120. If no, flow passes to 610, where the one or more processors of the central service center 102 report, such as to the regional system 104c, that the medical device is not registered in the regional system 104b. Accordingly, because the patient device is not found where expected, at 612, the one or more processors of the regional system 104c fail the device transfer request of the medical device. The device transfer request failure can be indicated at the clinician application 110c.

Returning to 608, if the medical device is found in the patient device database 116b, flow passes to 614 and the one or more processors of the regional system 104c perform an API call to query the global device index 120 to determine whether the medical device is currently found in any other regional system 104. In other embodiments, the search of the global device index 120 for all instances of the medical device may be accomplished at 606.

At 616, if the one or more processors of the central service center 102 determine that the medical device is found in any other region, flow passes the 618 and one or more processors of the central service center 102 report, such as to the regional system 104c and the clinician application 110c, that the medical device is registered in a different region. At 612, the one or more processors of the regional system 104c fail the device transfer request, because the device transfer request does not conform to the PDP requirements. This provides the technical advantage of preventing personal protected information from being transferring inappropriately.

Returning to 616, if the medical device is not found in any other region than the region requesting the transfer (e.g., regional system 104b), flow passes to 620 and the one or more processors of the regional system 104c register the medical device in the regional system 104c. In some cases, the medical device can only be registered if the medical device is first indicated as "pending" or "inactive" in the regional system 104b and the global device index 120.

At 622, the one or more processors of the regional system 104c perform an API call to the central service center 102 to request that the status indicator 212 of the global device index 120 be updated to register a record 202 associated with the unique ID(s) 204 entered at 604 to reflect that the status of the medical device is "active" in the regional system 104c and "inactive" in regional system 104b, as well as to update the region code 210.

At 624, the one or more processors of the central service center 102 transmit a communication of the regional system 104b to request that the patient device database 116b be updated from "pending" to "inactive".

At 626, the one or more processors of the central service center 102 complete the device transfer request, as needed. For example, the transfer of protected information (e.g., PHI, PII, etc.) between regions can be accomplished as discussed further below in at least Figure 10.

In other embodiments, once the patient device transfer request is entered, the one or more processors of the central service center 102 can perform at least some of the operations of determining where the patient device is registered, registering/unregistering the device, and completing the device transfer.

### Un-register a Medical Device

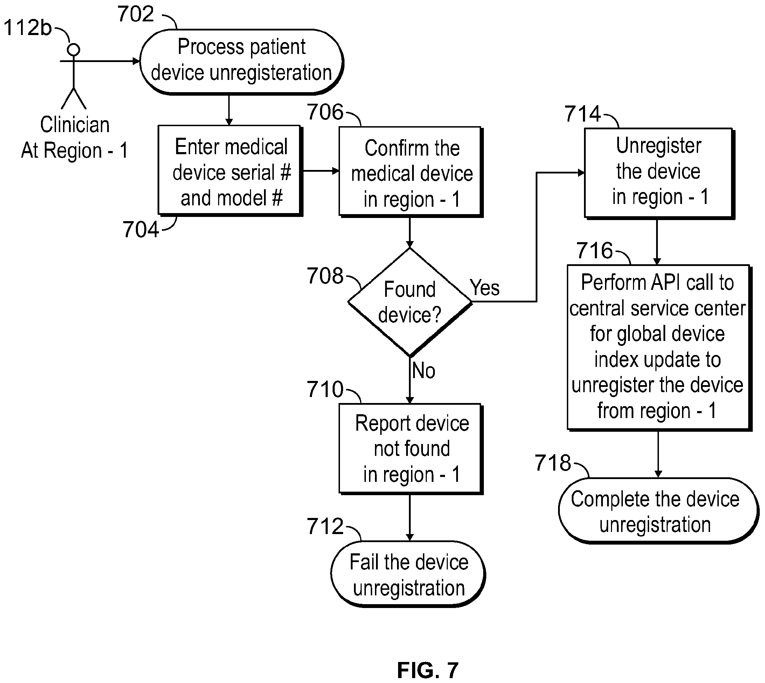

Figure 7 shows the process flow for un-registering a medical device in accordance with embodiments herein. The operations of Figure 7 may be implemented by hardware, firmware, circuitry and/or one or more processors housed partially and/or entirely within a local external device, remote server or more generally within a healthcare system. For example, the external devices/systems (e.g., local, remote or anywhere within the health care system) can include memory and one or more processors.

Figure 7 illustrates a clinician 112b within region 1, such as regional system 104b. At 702, one or more processors of the regional system 104b receive a request from the clinician application 110b to un-register a registered medical device. In some embodiments, the medical device is to be un-registered because the medical device may be removed, expired, replaced by a different medical device, patient deceased, available to be used by a different patient (e.g., external medical device), and the like. The un-registration process does not delete data from the central service center 102 or any of the regional systems 104. Instead, the process is a soft delete to set the status of the medical device to "inactive". Any associated patient data can be retained within the regional system 104, and, in some cases, archived, according to patient data requirements within the particular region.

At 704, the one or more processors of the regional system 104b receive, via the clinician application 110b, the unique ID(s) 204 associated with the medical device. In some embodiments, the device serial number 206 and the device model number 208 are received from the clinician application 110b.

At 706, the one or more processors of the regional system 104b search the patient device database 116b associated with regional system 104b based on the unique ID(s) 204 to confirm that the medical device is registered in the regional system 104b.

At 708, the one or more processors of the regional system 104b determine whether the patient device is entered into the patient device database 116b. If no, flow passes to 710, where the one or more processors of the regional system 104b report, such as to the clinician application 110b, that the medical device is not registered in the patient device database 116b. Accordingly, because the patient device is not registered, at 712, the one or more processors of the regional system 104b fail the un-registration of the medical device. The failure to un-register can be indicated at the clinician application 110b.

Returning to 708, if the medical device is found to be a registered device in the patient device database 116b, flow passes to 714 and the one or more processors of the regional system 104b un-register the medical device in the regional system 104b. In some embodiments, the status of the medical device is indicated as "inactive" in the patient device database 116b.

At 716, the one or more processors of the regional system 104b perform an API call to request that the central service center 102 update the global device index 120 to un-register the medical device from the regional system 104b.

At 718, the one or more processors of the central service center 102 complete the medical device un-registration, such as by changing the status of the medical device to "inactive". This synchronizes the data between the patient device database 116b and the global device index 120 to ensure the data is correct in both data storage locations (e.g., databases, etc.). In some cases, a confirmation communication of the un-registration can be sent to the regional system 104b and/or clinician application 110b.

In other embodiments, once the patient device unregistration request is entered, the one or more processors of the central service center 102 can perform at least some of the operations of confirming that the medical device is found in the expected region, unregistering the device, and completing the device unregistration.

### Deidentify Patient Identification in Cross-Region Operation

As discussed previously, PII and PHI must be protected at all times and not be shared across regions, and different regions have different regulatory standards (e.g., PDP requirements). In some cases, a patient can sign the appropriate documents to allow their PII and/or PHI to be shared, such as when the patient moves with the medical device to another region, visits a clinic/ medical facility in another region for care, and the like.

In general, common unique information (e.g., unique ID(s) 204, key(s), device model and serial number pair, etc.) non-PII and/or non-PHI can be identified across regions to determine whether the common unique information exists in any region, and where (e.g., in which region(s)) does the common unique information exist. The actions/methods described herein can be used to accomplish action(s) across regions using the common unique information, and without using or exposing the PII and/or PHI information.

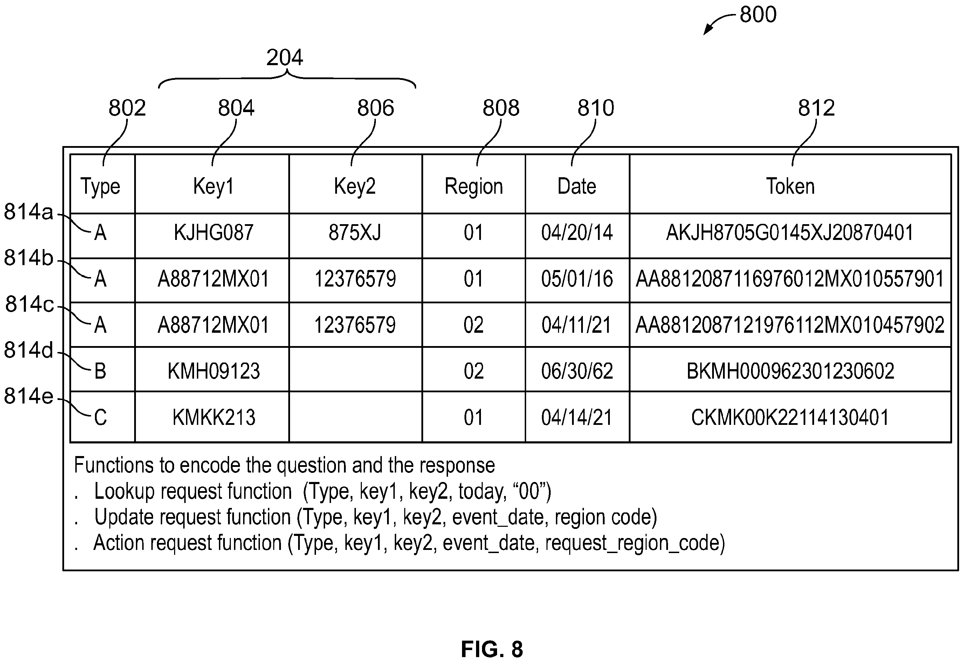

The central service center 102 provides consolidated area(s) for non-PII and/or non-PHI common unique information. The processes below discussed with respect to Figures 8-11 create API interfaces for processing requests. For example, API interfaces can be created for, but are not limited to, lookup requests from regions, update requests from regions, and action requests that can request cross-region action.

The inputs of requests must contain non-PII and/or non-PHI unique information (e.g., unique ID(s) 204). The data fields passing in as inputs should be encoded, and the encoded input can contain minimum "verification" information. The results or acknowledgement of requests, such as from the central service center 102 or the regional systems 104, can contain the verifiable request input, and can be a partial key input.

Figure 8 shows a table 800 of information used by the healthcare system 100 for global unique key mapping with encode functions in accordance with embodiments herein. Inputs can include some or all of the following, but are not limited to, type 802, key1 804, key2 806, region 808, date 810, and token 812. The token 812 can be used to calculate a function that encodes the query and, in some cases, the response. The function can include some or all of the data in 802, 804, 806, 808, and 810. To generate tokens 812, there can be multiple level surrogated keys, such as local surrogated key(s) and global surrogated key(s), for example. Global unique key mapping can be used with encoded response.

The type 802 can be used to indicate which request is input, such as lookup, update, transfer, etc. Although each type 802 in Figure 8 is indicated with a letter, other indicators such as numbers, strings, symbols, etc. can be used. Additionally, there can be less or more than three types 802.

Lines 814a, 814b, 814c, 814d, 814e of information to be encoded are shown. The lines 814a, 814b, and 814c indicate the use of two unique IDs, key1 804 and key2 806, that can be model and serial number, respectively. The lines 814b and 814c utilize the same unique key1 804 and key2 806 and thus are associated with the same medical device. The lines 814d and 814e each utilize a single unique ID 204.

### Preparing API Calls De-identified of PHI and PII

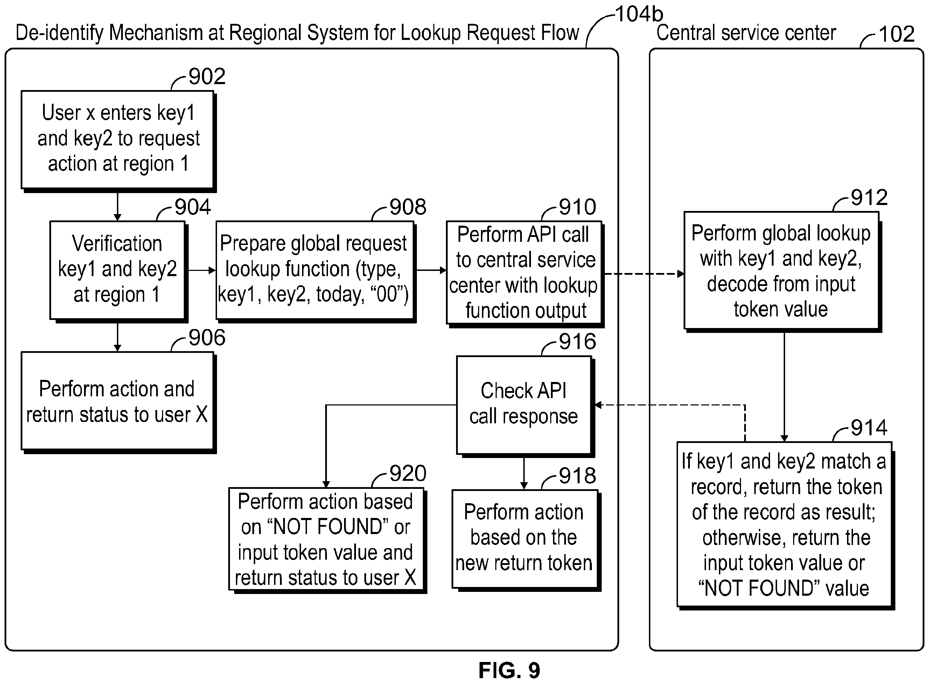

Figure 9 illustrates a process flow that utilizes one or more API call to lookup a medical device within the healthcare system 100 without exposing patient PHI or PII in accordance with embodiments herein. The operations of Figure 9 may be implemented by hardware, firmware, circuitry and/or one or more processors housed partially and/or entirely within a local external device, remote server or more generally within a healthcare system. For example, the external devices/systems (e.g., local, remote or anywhere within the health care system) can include memory and one or more processors.

The lookup function of Figure 9 could be used, for example, as part of the process flow for adding, registering, and/or changing a status of a medical device in a regional system 104. The regional system 104 de-identifies the data communications between the regional system 104 and the central service center 102 by using one or more unique ID 204 that is associated with a medical device, and the request does not include any PII and/or PHI information.

Although the regional system 104b is used as an example, any regional system 104 within the healthcare system 100 operates in a similar manner. At 902, one or more processors of the regional system 104b receive an input request, from the clinician application 110b, including one or more unique IDs 204 (e.g., key1 804 and key2 806). In some embodiments, at least two unique IDs 204 can be used, wherein one of the unique IDs 204 is associated with the medical device and one of the unique IDs 204 is associated with the patient, such as a birthdate. In other embodiments, one unique ID 204 or more than two unique IDs 204 can be used.

At 904, the one or more processors of the regional system 104b perform a local verification of the unique ID(s) 204 input at 902. The local verification can include a search of the patient device database 116b associated with regional system 104b based on the unique ID(s) 204 to confirm or deny that the medical device is registered in the regional system 104b.

In some embodiments, at 906, the one or more processors of the regional system 104b report, such as to the clinician application 110b, that the medical device is not registered in the patient device database 116b. This may be an expected result if the medical device is new. In other embodiments, if the medical device is being enrolled, the medical device may be added to the patient device database 116b with a status such as "pending" until the central service center 102 confirms that the medical device is not registered in any other regional system 104.

At 908, the one or more processors of the regional system 104b prepare a global request lookup function that includes the unique ID(s) 204 (e.g., key1 and key2 804, 806), and at least one of the type 802 of request (e.g., lookup, etc.), the date 810 (e.g., instant date, birthdate, etc.) and the region 808.

At 910, the one or more processors of the regional system 104b perform an API call to query the central service center 102 with the lookup function output to determine whether the medical device is currently "active" in any other region. The lookup function can be encoded using the token 812.

At 912, the one or more processors of the central service center 102 perform a global lookup with the received lookup function output that includes at least the one or more unique ID(s) 804, 806. The lookup function output can be decoded using the input token value of the token 812 to determine whether the one or more unique ID(s) match a record 202 (see Figure 2) in the global device index 120.

At 914, if a matching record 202 was found at 912, the one or more processors of the central service center 102 return the token value associated with the record 202 to the regional system 104b. If a matching record 202 was not found, the one or more processors of the central service center 102 return the input token value or a "NOT FOUND" value. It should be understood that other information can be transmitted from the central service center 102 to the regional system 104b to indicate a matching record found or no matching record found.

At 916, the one or more processors of the regional system 104b check the API call response (e.g., communication) transmitted by the central service center 102.

If the central service center 102 returned the token value of the matching record in the global device index 120 as a result at 916, flow passes to 918 where the one or more processors of the regional system 104b can output a message or report, such as to the clinician application 110b, that the medical device is registered in a different region, or that the medical device is already registered at the regional system 104b. In some embodiments, because the medical device is not allowed to be active in more than one region at a time, if the medical device is found in another region other than 104b, the one or more processors may indicate that the enrollment failed. In some cases, if the medical device already exists in a regional system 104, a message may be returned to the clinician application 110 to indicate the current status (e.g., open, active, inactive, etc.) of the medical device.

If the central service center 102 returned the input token value or a "NOT FOUND" value at 916, flow passes to 920 where the one or more processors of the regional system 104b can indicate the status to the clinician application 110. In some cases, a confirmation is provided to the clinician application 110 to advise the clinician 112 that the associated device is not already entered into the healthcare system 100 and thus is available to be registered to a patient.

Figure 10 illustrates a process flow that utilizes one or more API call to accomplish cross-region communications for requests that can also include moving patient data to another region without exposing patient PHI or PII in accordance with embodiments herein. The operations of Figure 10 may be implemented by hardware, firmware, circuitry and/or one or more processors housed partially and/or entirely within a local external device, remote server or more generally within a healthcare system. For example, the external devices/systems (e.g., local, remote or anywhere within the health care system) can include memory and one or more processors.

The cross-region communication of Figure 10 could be used, for example, to transfer (e.g., pull or push) patient data from one regional system 104 to another regional system 104 and update the global device index 120 and the patient device database(s) 116 appropriately. The regional system 104 de-identifies the data communications between the regional system 104 and the central service center 102 by using one or more unique ID 204 that is associated with a medical device, and the request does not include any PII or PHI information. Patient data is encrypted prior to moving the patient data from one region to another. It should be understood that the cross-region communication is not limited to transferring patient data, and that other utilization is contemplated.

Although the regional system 104b is used as an example, any regional system 104 within the healthcare system 100 operates in a similar manner. At 1002, one or more processors of the regional system 104b receive an input request, such as from the clinician application 110b, that includes one or more unique ID 204 (e.g., key1 804 and key2 806). The request can be to update a record associated with the one or more unique ID 204, a request to update a record and transfer patient data across regions, etc.

At 1004, the one or more processors of the regional system 104b perform a local verification of the unique ID(s) 204 input at 1002. The local verification can include a search of the patient device database 116b associated with regional system 104b based on the unique ID(s) 204 to confirm or deny that the medical device is registered in the regional system 104b. In some embodiments, the one or more processors of the regional system 104b can report, such as to the clinician application 11 0b, the registration status.

At 1006, the one or more processors of the regional system 104b prepare a global request action function that includes the unique ID(s) 804, 806, and at least one of the type 802 of request (e.g., transfer, etc.), the date 810 (e.g., instant date, birthdate, etc.) and the region 808 (e.g., the region where the medical device is currently enrolled, the region that the enrollment should be moved to, etc.).

At 1008, the one or more processors of the regional system 104b perform an API call to query the central service center 102 with the action function output to determine whether the medical device is currently "active" in any region. The action function can be encoded using the token 812. In some embodiments, the query can request to enroll, update, or request a cross-region transfer.

At 1010, the one or more processors of the central service center 102 perform a global lookup with the received action function output that includes at least the one or more unique ID(s) 204. The action function output can be decoded using the input token value of the token 812 to determine whether the one or more unique ID(s) 204 match a record 202 (see Figure 2) in the global device index 120.

At 1012, if a matching record 202 was found at 1010, the one or more processors of the central service center 102 return the token value associated with the record 202 to the regional system 104b. If a matching record 202 was not found, the one or more processors of the central service center 102 return the input token value or a "NOT FOUND" value.

At 1014, the one or more processors of the regional system 104b check the API call response (e.g., communication) transmitted by the central service center 102.

If the central service center 102 returned the input token value or a "NOT FOUND" value at 1014, flow passes to 1016 where the one or more processors of the regional system 104b can indicate the status to the clinician application 110. In some cases, a confirmation is provided to the clinician application 110 to advise the clinician 112 that the associated device is not entered into the healthcare system 100. Accordingly, a "transfer" request would fail, as the medical device is not available to transfer.

If the central service center 102 returned the token value of the matching record as a result as 1014, flow passes to 1018 where the one or more processors of the regional system 104b can perform the desired action based on the new return token value. If the needed information for a device and/or patient data transfer was included in the API call of 1008, the one or more processors of the regional system 104b may send a confirmation to perform the desired action. In other cases, the one or more processors can generate an API call or other formatted request to the central service center 102 with the cross-region request (e.g., to transfer the assignment of the medical device), in some cases along with patient data, to another region. In some embodiments, the request may specify what specific patient data to copy to the new region, such as a date-range of data, data associated with device settings, specify to copy all of the data, and the like.

At 1020, the one or more processors of the central service center 102 perform the cross-region action request. For example, a record 202 can be created or updated at the global device index 120, and/or patient data can be encrypted, and the encrypted patient data transmitted to another regional system 104. When patient data is transmitted, a copy of the patient data is sent. The original regional system 104 retains the patient data which may be archived. Also, the status of the medical device at the original regional system 104 is updated to "inactive", if needed, in the patient device database 116. Any data that is moved fulfills the PDP requirements.

At 1022, the one or more processors of the central service center 102 return the result of the action of 1020 to the regional system 104b.

At 1024, the one or more processors of the regional system 104b check the result of the cross-region action request and return the status to the user, such as through a message or indication on the clinician application 110. The status may be, for example, complete, approved, request failed, patient data successfully transferred, etc.

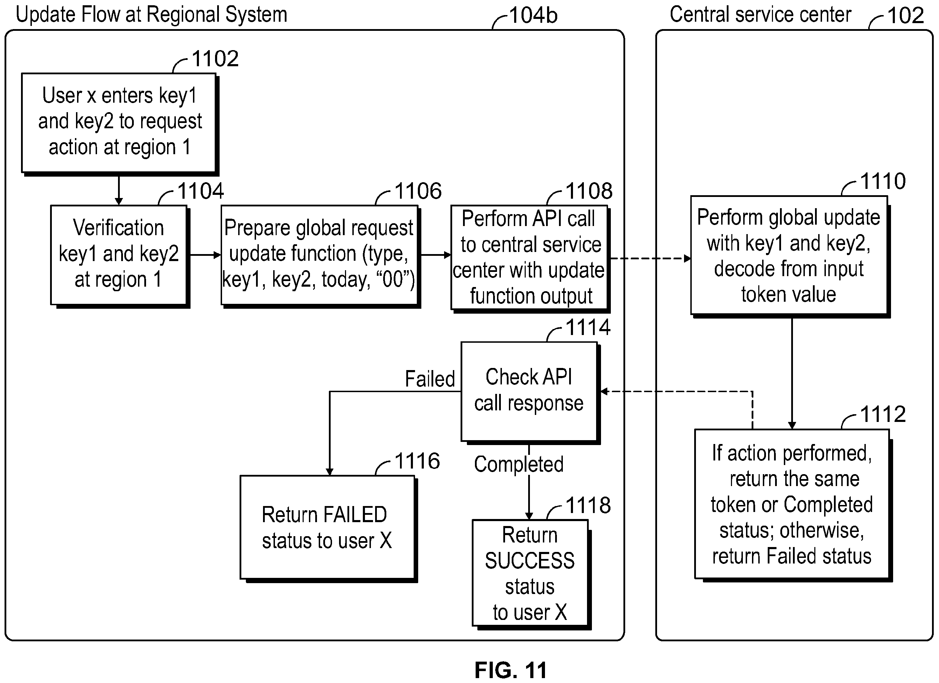

Figure 11 illustrates a process flow that utilizes one or more API call to update the global device index 120 to reflect a desired change within a regional system 104 without exposing patient PHI or PII in accordance with embodiments herein. The operations of Figure 11 may be implemented by hardware, firmware, circuitry and/or one or more processors housed partially and/or entirely within a local external device, remote server or more generally within a healthcare system. For example, the external devices/systems (e.g., local, remote or anywhere within the health care system) can include memory and one or more processors.

The update function of Figure 11 could be used, for example, to update the status of a device, such as to change the status from "active" to "inactive", from "inactive" to "active", etc. Because the information related to each medical device has to be kept current across the healthcare system 100, a clinician 112 or patient 108 cannot change certain data in the regional system 104 only. The global device index 120 in the central service center 102 is the master, and thus includes all the current information associated with each medical device.

Although the regional system 104b is used as an example, any regional system 104 within the healthcare system 100 operates in a similar manner. At 1102, the one or more processors of the regional system 104b receive an input request, such as from the clinician application 110b, that includes one or more unique ID 204 (e.g., key1 804 and key2 806 are shown in Figure 11). The request can be, for example, to update a record associated with the one or more unique ID 204.

At 1104, the one or more processors of the regional system 104b perform a local verification of the unique ID(s) 204 input at 1102. The local verification can include a search of the patient device database 116b associated with regional system 104b based on the unique ID(s) 204 to confirm or deny that the medical device is registered in the regional system 104b. In some embodiments, the one or more processors of the regional system 104b can report, such as to the clinician application 110b, the registration status.

At 1106, the one or more processors of the regional system 104b prepare a global request function that includes the unique ID(s) 204, and at least one of the type 802 of request (e.g., update, etc.), the date 810 (e.g., instant date, birthdate, etc.) and the region 808 (e.g., the region where the medical device is currently enrolled, etc.). For example, the global request can be to update the status of the medical device in the regional system 104b.

At 1108, the one or more processors of the regional system 104b perform an API call to the central service center 102 with the update function output of 1106 to request that the medical device be updated, such as to change the status indicator 212, in the global device index 120 and the patient device databases 116 of the regional systems 104. The update function can be encoded using the token 812.

At 1110, the one or more processors of the central service center 102 perform a global lookup with the received function output that includes at least the one or more unique ID(s) 804, 806. The string can be decoded using the input token value of the token 812 to determine whether the one or more unique ID(s) match a record 202 in the global device index 120. The one or more processors perform the global update to the global device index 120 if the desired update is allowed. For example, if the record 202 at the global device index 120 agrees with the record 202 (not shown) in the patient device database 116b, the one or more processors update the global device index 120. If the record 202 at the global device index 120 does not agree with the record 202 in the patient device database 116b, the global device index 120 is not updated. For example, if the medical device is "active" in another region, record is not found, or any other reason the data is in conflict with the request and cannot be updated, the global device index 120 is not updated.

At 1112, if the requested action was performed at 1110, the one or more processors of the central service center 102 transmit a communication / API call response to the regional system 104b that includes the same token value that was transmitted in 1108 or a "completed" status communication. If the action was not performed at 1110, the one or more processors transmit a "failed" status communication.

At 1114, the one or more processors of the regional system 104b check the API call response transmitted by the central service center 102.

If the central service center 102 returned a "failed" status communication at 1114, flow passes to 1116 where the one or more processors of the regional system 104 indicate the failed status of the request, such as to the clinician application 110b. In some cases, additional information related to the failure can be provided.

If the central service center 102 returned the original token value or a "completed" status communication at 1114, flow passes to 1118 where the one or more processors of the regional system 104 can output a message or report, such as to the clinician application 110b, to indicate that the requested transaction was successfully completed.

### Healthcare System

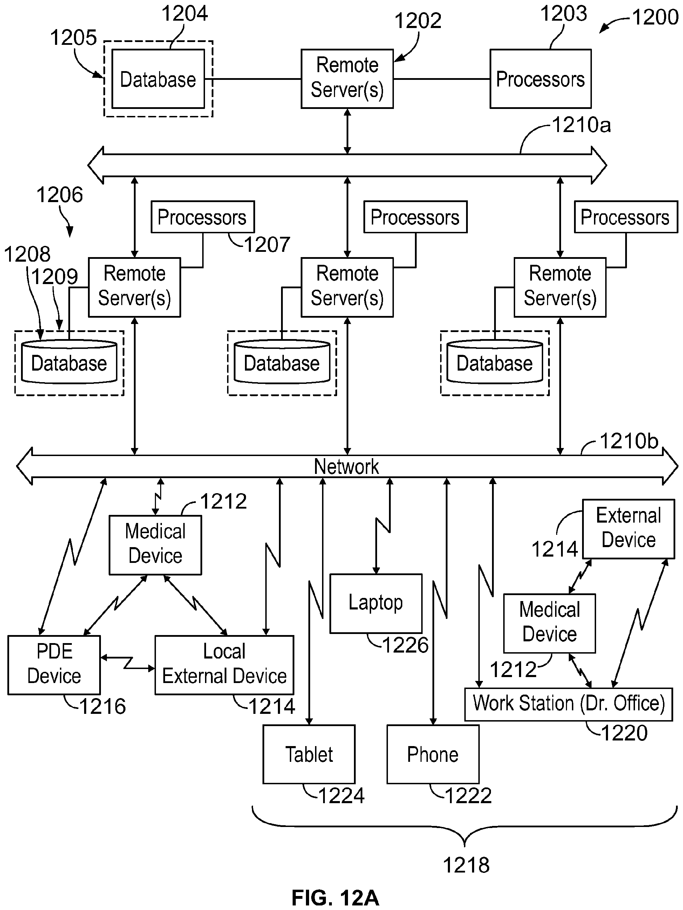

Figure 12A illustrates a healthcare system 1200 interfacing with a plurality of networked devices formed in accordance with embodiments herein. The healthcare system 1200 includes one or more servers 1202 and one or more processors 1203 connected to one or more database 1204 that is stored in one or more memory 1205, similar to the central service center 102 of Figure 1. The healthcare system 1200 further includes one or more servers 1206 and one or more processors 1207 connected to one or more database 1208 that is stored in one or more memory 1209, similar to the regional systems 104 of Figure 1, wherein each of the regional systems 104 can include one or more servers 1206, one or more processors 1207, and one or more database 1208 stored in one or more memory 1209. The servers 1206 and databases 1208 within a regional system 104, as well as the servers 1202 and databases 1204 of the central service center 102, can be located at a common physical location and/or distributed between multiple remote locations within a city, state, country, region, and/or the world, and/or the cloud. The one or more servers 1206 and one or more databases 1208 of different regional systems 104 can be located in different regions and/or different clouds.

The server(s) 1202 communicate with one or more network 1210a, and the servers 1206 communicate with one or more networks 1210a, 1210b. The network 1210 may be a private network, the internet, a voice over IP (VoIP) gateway, a local plain old telephone service (POTS) such as a public switched telephone network (PSTN), a cellular phone-based network, satellite-based network, a combination of one or more networks, and the like. Alternatively, the network 1210 may be a local area network (LAN), a campus area network (CAN), a metropolitan area network (MAN), or a wide area network (WAM). Although shown separately, the networks 1210a and 1210b can be connected to each other, such as to the internet. The network 1210b serves to provide a network that facilitates the transfer/receipt of information such as IMD data, other patient device data, and patient data such as PHI and PII.

The system 1200 also includes one or more medical devices 1212, one or more local external devices 1214, one or more patient data entry (PDE) devices 1216 and one or more medical personnel (MP) devices 1218, all of which communicate (directly or indirectly) through the network 1210b to the servers 1206 and/or one another. The medical device 1212 may be passive or active, may collect various types of data, such as cardiac electrical and/or mechanical activity data, PAP or other pressure related data, impedance data, RPM data, flow data, be an IMD, and the like. The PDE device 1216 collects data, such as based on manual inputs from a patient or other user, and/or based on automatic monitoring devices, such as the medical devices 1212 and/or local external devices 1214.

The local external device 1214 may be implemented as a variety of non-implantable devices including, but not limited to, a medical monitoring and/or medical services delivery device, BGA test devices, medical personnel programmer, a local RF transceiver and a user workstation, smart phone, tablet device, laptop computer, desktop computer and the like.

Accordingly, the medical devices 1212 and external devices 1214 deliver a particular treatment for a medical condition such as pacing, shocking, and the like for arrhythmia (e.g., VA, VT, AF, etc.) and/or other heart conditions, treatment of BGA conditions based on measurements of BGA test devices, and the like. Further, the medical devices 1212 and/or external devices 1214 deliver the particular treatment which transforms, such as in the case of arrhythmia, the patient's heart from an arrhythmia state to a normal sinus rhythm state.

The MP devices 1218 may also be implemented as a variety of devices including, but not limited to, medical personnel programmer, workstation 1220, smart phone 1222, tablet device 1224, laptop computer 1226, desktop computer and the like. Functionality of the MP devices 1218 related to embodiments herein may be implemented through dedicated hardware circuits, firmware, software, and/or application operating on one or more computing devices.

The server 1202 (e.g., central service center 102) controls the communication of information and requests between the servers 1206 including, but not limited to, at least some of patient data controlled by PDP requirements in one or more regions, IMD data, patient entered data, medical personnel entered data, such as to add a medical device 1212, move a medical device 1212 to or from another region, activate or inactivate a medical device 1212, and/or record information.

For example, in some embodiments, a healthcare system comprises a central service center 102 having memory 1205 and one or more processors 1203. The memory 1205 is configured to store program instructions and a global device index 120 that includes database records 202 associated with medical devices 1212. Each of the records 202 includes a corresponding unique device identifier (ID) 204, region code 210 and status indicator 212. The region code 210 is associated with a region having corresponding patient data privacy (PDP) requirements, and the status indicator 212 is indicative of whether the corresponding medical device 1212 is active in the corresponding region. The one or more processors 1203, when executing the program instructions, are configured to at least one of: i) manage transfer of patient data, for a first medical device 1212, between first and second regional systems 104 based on the PDP requirements for the corresponding first and second regional systems 104; ii) when the first and second regional systems 104 maintain corresponding status indicators 212 associated with a common medical device 1212, communicate with the first and second regional systems 104 to manage the status indicators 212, maintained by the first and second regional systems 104, associated with the common medical device 1212; iii) responsive to a registration request, for a candidate device, from the first regional system 104, return a registration response indicating either: a) the candidate device is registered in another regional system 104 or b) the candidate device is available for registration; or iv) responsive to an un-registration request, for a registered device, from the first regional system 104, update the status indicator 212 in the global device index 120 for the registered device in connection with the first regional system 104. In some embodiments, the unique ID includes at least one of i) a model number, ii) a serial number, iii) a unique key, iv) a second unique key, or v) a birthdate. In other embodiments, the global device index 120 is further configured to store, associated with the unique IDs, data that does not include patient information identified by the PDP requirements of the first and second regional systems 104.

In some embodiments, the one or more processors 1203 is configured to receive the registration request, for the candidate device, from the first regional system 104. The registration request includes one or more unique ID associated with the candidate device. Responsive to the registration request, the one or more processors 1203 is further configured to search, based on the one or more unique ID associated with the candidate device, the global device index 120 to determine whether the candidate device is included in any of the regional systems 104, and in response to determining that the one or more unique ID is included in the second regional system 104, transmit a communication to the first regional system 104 to report that the candidate device is included in the second regional system 104. In other embodiments, in response to determining that the one or more unique ID is not included in any of the regional systems 104, register, in the global device index 120, the first medical device 1212 in the first regional system 104.

In some embodiments, the one or more processors 1203 is configured to receive a transfer request from the first regional system 104, to transfer the first medical device 1212 from the first regional system 104 to the second regional system 104. The transfer request includes one or more unique ID associated with the first medical device 1212. Responsive to the transfer request, the one or more processors 1203 is further configured to search, based on the one or more unique ID associated with the first medical device 1212, the global device index 120 to determine whether the one or more unique ID is included in any of the regional systems 104, and in response to determining that the one or more unique ID is included in one of the regional systems 104 other than the first regional system 104, transmit a communication to the first regional system 104 to report that the first medical device 1212 is found in another regional system 104. In other embodiments, in response to determining that the one or more unique ID is not included in any of the regional systems 104 other than the first regional system 104, the one or more processors 1203 is configured to update, in the global device index 120, the first medical device 1212 to a pending status, and transmit a request to the second regional system 104 to transfer an assignment of the first medical device 1212 to the second regional system 104.

In some embodiments, the one or more processors 1203 is configured to receive a transfer request from the second regional system 104, to transfer the first medical device 1212 from the first regional system 104 to the second regional system 104. The transfer request includes one or more unique ID associated with the first medical device 1212. Responsive to the transfer request, the one or more processors 1203 is further configured to search, based on the one or more unique ID associated with the first medical device 1212, the global device index 120 to determine whether the one or more unique ID is included in any of the regional systems 104. In response to determining that the one or more unique ID is included in the first regional system 104 and is not included in any of the other regional systems 104, the one or more processors 1203 is configured to update the status indicator 212, in the global device index 120, to indicate an active status of the first medical device 1212 in the second regional system 104, and update the status indicator 212, in the global device index 120, to indicate an inactive status of the first medical device 1212 in the first regional system 104.

In some embodiments, the one or more processors 1203 is configured to receive a transfer request from the first regional system 104, to transfer the patient data associated with the first medical device 1212 from the first regional system 104 to the second regional system 104. The transfer request includes one or more unique ID associated with the first medical device 1212. Responsive to the transfer request, the one or more processors 1203 is further configured to, in response to approving the transfer request, transmit a communication to the first regional system 104 to request that the first regional system 104 transmit the patient data to the second region, wherein the patient data includes at least one piece of protected information identified by the corresponding PDP requirements. In some embodiments, the patient data is encrypted.

In some embodiments, the one or more processors 1203 is configured to receive a request from the first regional system 104. The request includes an input token value, and the request is one of the registration request, the un-registration request, an update request, or a transfer request. The request includes one or more unique ID 204 associated with one of the medical devices 1212. Responsive to the request, the one or more processors 1203 is further configured to decode the request using the input token value, and return, to the first regional system 104, at least one of a request failed value, a request completed value, a token value associated with a record 202 in the global device index 120, the input token value, a not found value, a completed value, or a failed value.

In some embodiments, a healthcare system comprises a first regional system 104 that comprises a memory 1209 and one or more processors 1207. The memory 1209 is configured to store program instructions and a regional patient device database 116 including database records 202 associated with medical devices 1212. Each of the records 202 includes a corresponding unique device identifier (ID) 204 and status indicator 212. The regional patient device database 116 is associated with a region having corresponding patient data privacy (PDP) requirements, and the status indicator 212 is indicative of whether the corresponding medical device 1212 is active in the corresponding region. The one or more processors 1207 that, when executing the program instructions, is configured to at least one of: i) transmit or receive patient data, for a first medical device 1212, to or from a second regional system 104 associated with a second region, the patient data based on the PDP requirements for at least one of the first and second regional systems 104; ii) responsive to an update request, for a registered device 1212, update the status indicator 212 for the registered device1212; iii) responsive to a registration request, for a candidate device 1212, fail the registration of the candidate device 1212 if the candidate device 1212 is registered in the regional patient device database 116; or iv) responsive to an un-registration request, for a registered device 1212, un-register the registered device 1212 in the regional patient device database 116.

In some embodiments, the one or more processors 1207 is configured to transmit a request to a central service center 102, the request being one of the registration request, the un-registration request, the update request, or a transfer request. The request includes one or more unique ID 204 associated with one of the medical devices 1212 and an input token value 812.

In some embodiments, the one or more processors 1207 is configured to: i) determine that the candidate device1212 is not registered in the regional patient device database 116; ii) transmit the registration request to a central service center 102, the registration request including one or more unique ID 204 associated with candidate device 1212; and iii) responsive to a communication indicating that the candidate device 1212 is not registered in the second regional system 104, register the candidate device 1212 in the regional patient device database 116.

In some embodiments, the one or more processors 1207 is configured to i) transmit a transfer request to a central service center 102 to request to transfer the first medical device 1212 from the first regional system 104 to the second regional system 104, wherein the transfer request includes one or more unique ID 204 associated with the first medical device 1212; and ii) responsive to a communication indicating that the first medical device 1212 is found in the second regional system 104, fail the transfer request.

In some embodiments, the one or more processors 1207 is configured to: i) transmit a transfer request to a central service center 102 to request to transfer a medical device 1212 from the second regional system 104 to the first regional system 104, wherein the transfer request includes one or more unique ID 204 associated with the medical device 1212; and ii) responsive to a communication indicating that medical device 1212 is found in the second regional system 104, register the medical device 1212 in the first regional system 104.

Other servers and memory systems (not shown) can receive data from the devices for storage, retrieval, data collection, data analysis, diagnosis, treatment recommendations and the like. Other databases and file structures (not shown) store all or various portions of the information described herein, including, but not limited to, IMD data, medical record information, treatment diagnoses and recommendations, and the like. The local external device 1214 may reside in a patient's home, a hospital, or a physician's office, or be interconnected to a patient's skin surface. The local external device 1214 communicates wired or wirelessly with one or more medical device 1212 and/or the network 1210b. The servers and their associated devices described herein may wirelessly communicate with one another utilizing various protocols, such as Bluetooth, GSM, infrared wireless LANs, HIPERLAN, 3G, satellite, as well as circuit and packet data protocols, and the like. Alternatively, a hard-wired connection may be used to connect the servers and devices. The local external device 1214, when implemented as a programmer, may be configured to acquire cardiac signals from the surface of a person (e.g., ECGs), and/or intra-cardiac electrogram (e.g., IEGM) signals from the medical device 1212. The local external device 1214 interfaces with the network 121 0b to upload the data and other information to the appropriate server. Optionally, the local external device 1214 may represent a local RF transceiver that interfaces with the network 1210b to upload IMD data and/or BGA data.

The workstation 1220 may interface with the network 1210b via the internet or POTS to download various data, information, diagnoses, and treatment recommendations from the database 1208. Alternatively, the workstation 1220 may download raw data from the surface ECG units, leads, or monitoring device via either the programmer or the local RF transceiver. Once the user workstation 1220 has downloaded the cardiac signal waveforms, ventricular and atrial heart rates, or detection thresholds, and the like, the user workstation 1220 may process the information. The system may download information and notifications to the smart phone 1222, the tablet device 1224, the laptop computer 1226, or to a server to be stored on a database.

**Thus,** provided is a distributed "digital" healthcare system that collects various types of data, enables the data to be analyzed by various computing devices within the system and securely facilitates the transfer of private patient data from one region to another region, while adhering to the applicable PDP requirements. Advantages of the system include but are not limited to securing the privacy of the data as the patient data is prepared by the central service center 102 and/or regional systems 104, encrypted, and transmitted via the central service center 102. If changes are made to any of the regions' PDP regulations and/or requirements, the servers 1202 and 1206 can be updated at a network level. A patient's personal data is not exposed as no person is involved in facilitating the transfer of the actual data between regions. Further, status and location of each medical device 1212 is tracked by the central service center, allowing a patient who wishes to move their data from one region to another to have one secure point of contact, such as a doctor's office or hospital, to request the transfer. The central service center 102 also prohibits the same medical device 1212 from being active in different regions, resulting in improved continuance of care. Thus, an improved system and methodology are provided.

In some embodiments, the IMD data and medical data associated with a medical device 1212 located in a first region (e.g., region 1) can be transferred to a second region (e.g., region 2) when the patient device / medical device 1212 is reassigned from the region system 104b to the regional system 104c. For example, the patient may have moved to the second region and wishes to receive their medical care within the second region. The system therefore transforms the patient data to a different state, one in which the patient data and the medical device 1212 can only be accessed by clinicians within one particular assigned region.

The transfer of the patient data imposes meaningful limits on how patient data can be used and who can access the data, as well as who can provide treatment, by limiting the access and treatment to specific clinicians, etc., within the current region (e.g., second region). Treatment can include, but is not limited to, program and/or reprogram the medical device 1212, such as by adjusting settings and/or parameters on the medical device 1212, updating software on the medical device 1212, and delivering a particular treatment for medical conditions via the medical device 1212 (e.g., to treat arrhythmia, heart rate, hemodynamic instability, blood glucose levels). The treatment can transform the patient's heart, such as from an arrhythmia state to a normal sinus rhythm state. The treatment can be modified to transform the patient and/or patient symptoms to a desired state by adjusting settings of the medical device 1212 as the patient's condition changes, such as in response to monitoring heart sounds, CA signals, posture, heart rate, hemodynamic stability, blood glucose levels, etc.

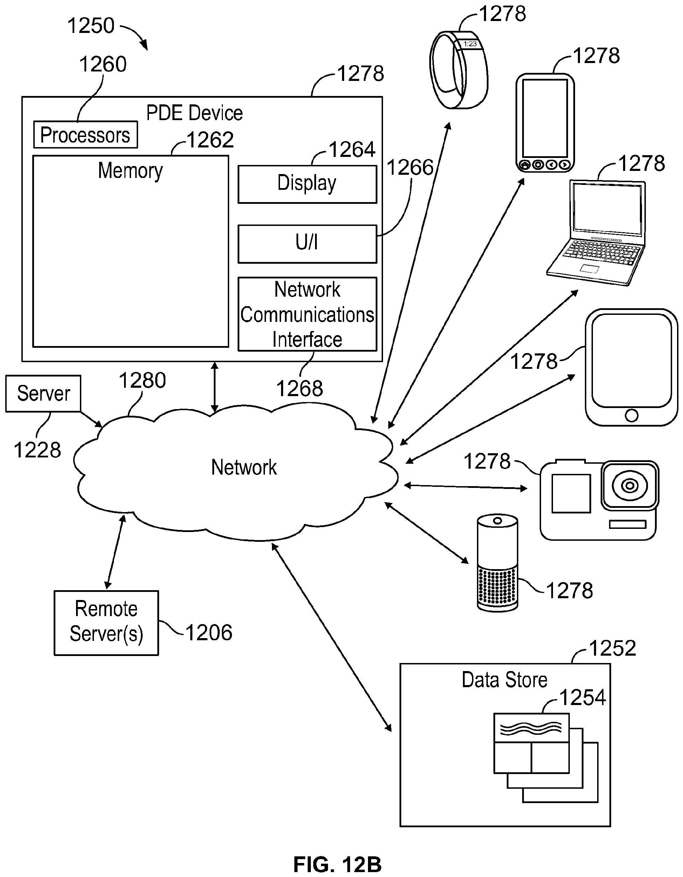

Figure 12B illustrates a distributed healthcare system 1250 that can be included in one of the regional systems 104 in accordance with embodiments herein. The system 1250 can collect and analyze patient data, and communicate over a network 1280 with one or more server 1228 that can in turn communicate with server(s) 1206 within the regional system 104. The system 1250 includes one or more patient data entry (PDE) devices 1278 that communicate over the network 1280 with various other devices, such as IMDs, body generated analyte (BGA) test devices, MP devices, local external devices, servers and the like. Optionally, the PDE devices 1278 may communicate through a wholly or partially wired subsystem. The network 1280 may represent the World Wide Web, a local area network, a wide area network and the like. When the PDE device 1278 includes a GUI, the patient or other user may input patient data in addition to IMD data, internal and external medical device data, and BGA data. Optionally, the PDE devices 1278 may include one or more microphones that are configured to listen for audible information spoken by a user or patient, such as a verbal statement to enter patient data. Optionally, the PDE devices 1278 may include one or more cameras that are configured to capture still images and/or video that is processed utilizing image recognition to identify what action a patient is performing (e.g., what, when and how much a patient is eating and/or drinking). The PDE device 1278 includes one or more processors 1260, memory 1262, a display 1264, a user interface 1266, a network communications interface 1268, and various other mechanical components, electrical circuits, hardware and software to support operation of the PDE device 1278. It is recognized that not all PDE devices 1278 include a display, user interface, and the like. For example, a fixed or handheld camera may simply include camera related electronics and network circuitry to support communication to and from the camera.

**The** user interface 1266 is configured to receive information from a patient or clinician. The user interface 1266 may include a variety of visual, audio, and/or mechanical devices. For example, the user interface 1266 can include a visual input device such as an optical sensor or camera, an audio input device such as a microphone, and a mechanical input device such as a keyboard, keypad, selection hard and/or soft buttons, switch, touchpad, touch screen, icons on a touch screen, touch sensitive areas on a touch sensitive screen and/or any combination thereof. Similarly, the user interface 1266 can include a visual output device such as a liquid crystal display screen, one or more light emitting diode indicators, an audible output device such as a speaker, alarm and/or buzzer, and a mechanical output device such as a vibrating mechanism. The display may be touch sensitive to various types of touch and gestures. As further examples, the user interface 1266 may include a touch sensitive screen, a non-touch sensitive screen, a text-only display, a smart phone display, an audible output (e.g., a speaker or headphone jack), and/or any combination thereof. The user interface 1266 permits the user to select one or more of a switch, button or icon in connection with various operations of the PDE device 1278.

The memory 1262 can encompass one or more memory devices of any of a variety of forms (e.g., read only memory, random access memory, static random-access memory, dynamic random access memory, etc.) and can be used by the processor 1260 to store and retrieve data. The data that is stored by the memory 1262 can include, but need not be limited to, operating systems, applications, and other information. Each operating system includes executable code that controls basic functions of the communication device, such as interaction among the various components, communication with external devices via a wireless transceivers and/or component interface, and storage and retrieval of applications and data to and from the memory 1262. Each application includes executable code that utilizes an operating system to provide more specific functionality for the communication devices, such as file system service and handling of protected and unprotected data stored in the memory 1262.

The network communications interface 1268 provides a direct connection to other devices, auxiliary components, or accessories for additional or enhanced functionality, and in particular, can include a USB port for linking to a user device with a USB cable. Optionally, the network communications interface 1268 may include one or more transceivers that utilize a known wireless technology for communication.

The memory 1252 may store the object catalogs 1254 organized in various manners and related to a wide variety of objects and types of data. The object catalogs 1254 may be organized and maintained within any manner of data sources, such as data bases, text files, data structures, libraries, relational files, flat files and the like. The object catalogs 1254 include various types of templates corresponding to different types of objects.

### Closing Statements

It should be clearly understood that the various arrangements and processes broadly described and illustrated with respect to the Figures, and/or one or more individual components or elements of such arrangements and/or one or more process operations associated of such processes, can be employed independently from or together with one or more other components, elements and/or process operations described and illustrated herein. Accordingly, while various arrangements and processes are broadly contemplated, described and illustrated herein, it should be understood that they are provided merely in illustrative and non-restrictive fashion, and furthermore can be regarded as but mere examples of possible working environments in which one or more arrangements or processes may function or operate.

**As** will be appreciated by one skilled in the art, various aspects may be embodied as a system, method or computer (device) program product. Accordingly, aspects may take the form of an entirely hardware embodiment or an embodiment including hardware and software that may all generally be referred to herein as a "circuit," "module" or "system." Furthermore, aspects may take the form of a computer (device) program product embodied in one or more computer (device) readable storage medium(s) having computer (device) readable program code embodied thereon.

Any combination of one or more non-signal computer (device) readable medium(s) may be utilized. The non-signal medium may be a storage medium. A storage medium may be, for example, an electronic, magnetic, optical, electromagnetic, infrared, or semiconductor system, apparatus, or device, or any suitable combination of the foregoing. More specific examples of a storage medium would include the following: a portable computer diskette, a hard disk, a random access memory (RAM), a dynamic random access memory (DRAM), a read-only memory (ROM), an erasable programmable read-only memory (EPROM or Flash memory), a portable compact disc read-only memory (CD-ROM), an optical storage device, a magnetic storage device, or any suitable combination of the foregoing.

Program code for carrying out operations may be written in any combination of one or more programming languages. The program code may execute entirely on a single device, partly on a single device, as a stand-alone software package, partly on single device and partly on another device, or entirely on the other device. In some cases, the devices may be connected through any type of network, including a local area network (LAN) or a wide area network (WAN), or the connection may be made through other devices (for example, through the Internet using an Internet Service Provider) or through a hard wire connection, such as over a USB connection. For example, a server having a first processor, a network interface, and a storage device for storing code may store the program code for carrying out the operations and provide this code through its network interface via a network to a second device having a second processor for execution of the code on the second device.

Aspects are described herein with reference to the Figures, which illustrate example methods, devices and program products according to various example embodiments. These program instructions may be provided to a processor of a general purpose computer, special purpose computer, or other programmable data processing device or information handling device to produce a machine, such that the instructions, which execute via a processor of the device implement the functions/acts specified. The program instructions may also be stored in a device readable medium that can direct a device to function in a particular manner, such that the instructions stored in the device readable medium produce an article of manufacture including instructions which implement the function/act specified. The program instructions may also be loaded onto a device to cause a series of operational steps to be performed on the device to produce a device implemented process such that the instructions which execute on the device provide processes for implementing the functions/acts specified.

**The** units, modules, applications herein may include any processor-based or microprocessor-based system including systems using microcontrollers, reduced instruction set computers (RISC), application specific integrated circuits (ASICs), field-programmable gate arrays (FPGAs), logic circuits, and any other circuit or processor capable of executing the functions described herein. Additionally or alternatively, the modules/controllers herein may represent circuit modules that may be implemented as hardware with associated instructions (for example, software stored on a tangible and non-transitory computer readable storage medium, such as a computer hard drive, ROM, RAM, or the like) that perform the operations described herein. The above examples are exemplary only and are thus not intended to limit in any way the definition and/or meaning of the term "controller." The units/modules/applications herein may execute a set of instructions that are stored in one or more storage elements, in order to process data. The storage elements may also store data or other information as desired or needed. The storage element may be in the form of an information source or a physical memory element within the modules/controllers herein. The set of instructions may include various commands that instruct the modules/applications herein to perform specific operations such as the methods and processes of the various embodiments of the subject matter described herein. The set of instructions may be in the form of a software program. The software may be in various forms such as system software or application software. Further, the software may be in the form of a collection of separate programs or modules, a program module within a larger program or a portion of a program module. The software also may include modular programming in the form of object-oriented programming. The processing of input data by the processing machine may be in response to user commands, or in response to results of previous processing, or in response to a request made by another processing machine.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

It is to be understood that the above description is intended to be illustrative, and not restrictive. For example, the above-described embodiments (and/or aspects thereof) may be used in combination with each other. In addition, many modifications may be made to adapt a particular situation or material to the teachings herein without departing from its scope. While the dimensions, types of materials and coatings described herein are intended to define various parameters, they are by no means limiting and are illustrative in nature. Many other embodiments will be apparent to those of skill in the art upon reviewing the above description. The scope of the embodiments should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects or order of execution on their acts.

## Claims

1. A healthcare system (100, 1200) comprising:
a central service center (102) comprising:
memory (1205) configured to store program instructions and a global device index (120) including database records (202a, 202b, 202c) associated with medical devices (1212), each database record (202a, 202b, 202c) including a corresponding unique device identifier (ID) (204), region code (210) and status indicator (212), the region code (210) associated with a region having corresponding patient data privacy (PDP) requirements, the status indicator (212) indicative of whether the corresponding medical device (1212) is active in the corresponding region; and
one or more processors (1203) that, when executing the program instructions, are configured to at least one of:
manage transfer of patient data, for a first medical device (1212), between first and second regional systems (104a, 104b) based on the PDP requirements for the corresponding first and second regional systems (104a, 104b);
when the first and second regional systems (104a, 104b) maintain corresponding status indicators (212) associated with a common medical device (1212), communicate with the first and second regional systems (104a, 104b) to manage the status indicators (212), maintained by the first and second regional systems (104a, 104b), associated with the common medical device (1212);
responsive to a registration request, for a candidate device, from the first regional system (104a, 104b), return a registration response indicating either: i) the candidate device is registered in another regional system (104b, 104c) or ii) the candidate device is available for registration; or
responsive to an un-registration request, for a registered device, from the first regional system (104a), update the status indicator (212) in the global device index (120) for the registered device in connection with the first regional system (104a).

2. The system of claim 1, wherein the unique ID (204) includes at least one of i) a model number (206), ii) a serial number (208), iii) a unique key, iv) a second unique key, or v) a birthdate.

3. The system of claim 1 or 2, wherein the global device index (120) is further configured to store, associated with the unique IDs (204), data that does not include patient information identified by the PDP requirements of the first and second regional systems (104a, 104b, 104c).

4. The system of any one of claims 1 to 3, wherein the one or more processors (1203) is configured to receive the registration request, for the candidate device, from the first regional system (104a), wherein the registration request includes one or more unique ID (204) associated with the candidate device, and responsive thereto, the one or more processors (1203) is further configured to:
search, based on the one or more unique ID (204) associated with the candidate device, the global device index (120) to determine whether the candidate device is included in any of the regional systems; and
in response to the determination that:
i) the one or more unique ID (204) is included in the second regional system (104b), transmit a communication to the first regional system (104a) to report that the candidate device is included in the second regional system (104b); or
ii) the one or more unique ID (204) is not included in any of the regional systems (104a, 104b, 104c), register, in the global device index (120), the first medical device (1212) in the first regional system (104a).

5. The system of any one of claims 1 to 4, wherein the one or more processors (1203) is configured to receive a transfer request from the first regional system (104a), to transfer the first medical device (1212) from the first regional system (104a) to the second regional system (104b), wherein the transfer request includes one or more unique ID (204) associated with the first medical device (1212), and responsive thereto, the one or more processors (1203) is further configured to:
search, based on the one or more unique ID (204) associated with the first medical device (1212), the global device index (120) to determine whether the one or more unique ID (204) is included in any of the regional systems (104a, 104b, 104c); and
in response to the determination that:
i) the one or more unique ID ("04) is included in one of the regional systems (104b, 104c) other than the first regional system (104a), transmit a communication to the first regional system (104a) to report that the first medical device (1212) is found in another regional system (104a, 104b); or
ii) the one or more unique ID (204) is not included in any of the regional systems (104b, 104c) other than the first regional system (104a):
ii-a) update, in the global device index (120), the first medical device (1212) to a pending status; and
ii-b) transmit a request to the second regional system (104b) to transfer an assignment of the first medical device (1212) to the second regional system (104b).

6. The system of any one of claims 1 to 5, wherein the one or more processors (1203) is configured to receive a transfer request from the second regional system (104b), to transfer the first medical device (1212) from the first regional system (104a) to the second regional system (104b), wherein the transfer request includes one or more unique ID (204) associated with the first medical device (1212), and responsive thereto, the one or more processors (1203) is further configured to:
search, based on the one or more unique ID (204) associated with the first medical device (1212), the global device index (!20) to determine whether the one or more unique ID (204) is included in any of the regional systems (104a, 104b, 104c); and
in response to the determination that the one or more unique ID (204) is included in the first regional system (104a) and is not included in any of the other regional systems (104b, 104c),
update the status indicator (212), in the global device index (120), to indicate an active status of the first medical device (1212) in the second regional system (104b); and
update the status indicator (212), in the global device index (120), to indicate an inactive status of the first medical device (1212) in the first regional system (104a).

7. The system of any one of claims 1 to 6, wherein the one or more processors (1203) is configured to receive a transfer request from the first regional system (104a), to transfer the patient data associated with the first medical device (1212) from the first regional system (104a) to the second regional system (104b), wherein the transfer request includes one or more unique ID (204) associated with the first medical device (1212), and responsive thereto, the one or more processors (1203) is further configured to:
in response to approval of the transfer request, transmit a communication to the first regional system (104a) to request that the first regional system (104a) transmit the patient data to the second regional system (104b), wherein the patient data includes at least one piece of protected information identified by the corresponding PDP requirements, preferably wherein the patient data is encrypted.

8. The system of any one of claims 1 to 7, wherein the one or more processors (1203) is configured to receive a request from the first regional system (104a), wherein the request includes an input token value, the request being one of the registration request, the un-registration request, an update request, or a transfer request, wherein the request includes one or more unique ID (204) associated with one of the medical devices (1212), and responsive thereto, the one or more processors (1203) is further configured to:
decode the request using the input token value; and
return, to the first regional system (104a), at least one of a request failed value, a request completed value, a token value associated with a record in the global device index, the input token value, a not found value, a completed value, or a failed value.

9. A computer implemented method comprising:
storing, in memory (1205), a global device index (120) that comprises database records (202a, 202b, 202c) associated with medical devices (1212), each database record (202a, 202b, 202c) including a corresponding unique device identifier (ID) (204), region code (210) and status indicator (212), the region code (210) associated with a region having corresponding patient data privacy (PDP) requirements, the status indicator(212) indicative of whether the corresponding medical device (1212) is active in the corresponding region; and
utilizing one or more processors (1203) that, when executing specific program instructions, perform at least one of:
managing, utilizing one or more processors (1203) of a central service center (102), a transfer of patient data, for a first medical device (1212), between first and second regional systems (104a, 104b) based on the PDP requirements for the corresponding first and second regional systems (104a, 104b);
when the first and second regional systems (104a, 104b) maintain corresponding status indicators (212) associated with a common medical device (1212), communicating, utilizing the one or more processors (1203), with the first and second regional systems (104a, 104b) to manage the status indicators (212), maintained by the first and second regional systems (104a, 104b), associated with the common medical device (1212);
responsive to a registration request, for a candidate device (1212), from the first regional system (104a), returning, utilizing the one or more processors (1203), a registration response indicating either: i) the candidate device (1212) is registered in another regional system (104b, 104c) or ii) the candidate device (1212) is available for registration; or
responsive to an un-registration request, for a registered device, from the first regional system (104a), updating, utilizing the one or more processors (1203), the status indicator (212) in the global device index (120) for the registered device in connection with the first regional system (104).

10. The method of claim 9, wherein in response to receiving, utilizing the one or more processors (1203), a transfer request from the first regional system (104a) requesting to transfer registration of the first medical device (1212) from the first regional system (104a) to the second regional system (104b), wherein the transfer request includes one or more unique ID (204) associated with the first medical device (1212), the managing the transfer of patient data further comprises:
searching, utilizing the one or more processors (1203), based on the one or more unique ID (204) associated with the first medical device (1212), the global device index (120) to determine whether the one or more unique ID (204) is included in any of the regional systems (104a, 104b, 104c); and
in response to determining, utilizing the one or more processors (1203), that:
i) the first medical device (1212) is not included in any of the regional systems (104b, 104c) other than the first regional system (104a):
i-a) updating, utilizing the one or more processors (1203), the status indicator (212) of the database record (202a, 202b, 202c) in the global device index (120) associated with the at least one unique ID (204) to a pending status; and
i-b) transmitting, utilizing the one or more processors (1203), a transfer request associated with the first medical device (1212) to the second regional system (104b); or
ii) the first medical device (1212) is included in the second regional system (104b) and is not included in any of the other regional systems (104a, 104b):
ii-a) updating, utilizing the one or more processors (1203), the status indicator (212) of the database record (202a, 202b, 202c) in the global device index (120) associated with the at least one unique ID (204) to register the first medical device (1212) in the first regional system (104a); and
ii-b) transmitting, utilizing the one or more processors (1203), an un-registration request associated with the first medical device (1212) to the second regional system (104b).

11. A healthcare system (100, 1200) comprising:
a first regional system (104a), comprising:
memory (1209) configured to store program instructions and a regional patient device database (1208) including database records (202a, 202b, 220c) associated with medical devices (1212), each database record (202a, 202b, 202c) including a corresponding unique device identifier (ID) (204) and status indicator (212), the regional patient device database (1208) associated with a region having corresponding patient data privacy (PDP) requirements, the status indicator (212) indicative of whether the corresponding medical device (1212) is active in the corresponding region; and
one or more processors (1207) that, when executing the program instructions, is configured to at least one of:
transmit or receive patient data, for a first medical device (1212), to or from a second regional system (104b) associated with a second region, the patient data based on the PDP requirements for at least one of the first and second regional systems (104a, 104b);
responsive to an update request, for a registered device, update the status indicator (212) for the registered device;
responsive to a registration request, for a candidate device, fail the registration of the candidate device if the candidate device is registered in the regional patient device database (1208); or
responsive to an un-registration request, for a registered device, un-register the registered device in the regional patient device database (1208).

12. The system of claim 11, wherein the one or more processors (1207) is configured to transmit a request to a central service center (102), the request being one of the registration request, the un-registration request, the update request, or a transfer request, wherein the request includes one or more unique ID (204) associated with one of the medical devices (1212), wherein the request includes an input token value.

13. The system of claim 11 or 12, wherein the one or more processors (1207) is configured to:
determine that the candidate device is not registered in the regional patient device database (1208);
transmit the registration request to a central service center (102), the registration request including one or more unique ID (204) associated with candidate device; and
responsive to a communication indicating that the candidate device is not registered in the second regional system (104b), register the candidate device in the regional patient device database (1208).

14. The system of any one of claims 11 to 13, wherein the one or more processors (1207) is configured to:
transmit a transfer request to a central service center (102) to request to transfer the first medical device (1212) from the first regional system (104a) to the second regional system (104b), wherein the transfer request includes one or more unique ID (204) associated with the first medical device (1212); and
responsive to a communication indicating that the first medical device (1212) is found in the second regional system (104b), fail the transfer request.

15. The system of any one of claims 11 to 14, wherein the one or more processors (1207) is configured to:
transmit a transfer request to a central service center (102) to request to transfer a medical device (1212) from the second regional system (104b) to the first regional system (104a), wherein the transfer request includes one or more unique ID (204) associated with the medical device (1212); and
responsive to a communication indicating that medical device (1212) is found in the second regional system (104b), register the medical device (1212) in the first regional system (104a).
